# EUROPÄISCHE PATENTSCHRIFT

(11) **EP 0 755 929 B1**
(45) Veröffentlichungstag und Bekanntmachung des Hinweises auf die Patenterteilung: **27.03.2002**
(21) Anmeldenummer: 96111562.3
(22) Anmeldetag: 18.07.1996
(51) Int. Cl.: C07D 319/12, C09K 19/34

(54) **Photovernetzbare flüssigkristalline 1,4-Dioxan-2,3-diyl-Derivate**
Photocrosslinkable liquid crystalline 1,4-dioxane-2,3-diyl derivatives
Dérivés photoréticulables du 1,4-dioxane-2,3-diyl ayant des propriétés de cristaux liquides

(30) Priorität: 28.07.1995 CH 221995; 09.05.1996 EP 96107342
(43) Veröffentlichungstag der Anmeldung: 29.01.1997
(73) Patentinhaber: Rolic AG, 6301 Zug (CH)
(72) Erfinder: Kelly, Stephen, Beverley, East Yorkshire HU17 8XA (GB)
(74) Vertreter: Liebetanz, Michael, Dipl.-Phys.

(56) Entgegenhaltungen:
- EP-A- 0 630 892
- WO-A-95/16007
- GB-A- 2 142 020

## Beschreibung

Die vorliegende Erfindung betrifft photovernetzbare flüssigkristalline 1,4-Dioxan-2,3-diyl-Derivate, flüssigkristalline Gemische, die solche Verbindungen enthalten, sowie deren Verwendung im vernetzten Zustand als optische Bauelemente.

Durch geeignete Orientierungsschichten oder in einem Feld können photovernetzbare Flüssigkristalle, welche mit einer geeigneten Menge eines Photoinitiators versehen sind, auf einem Substrat oder in einer Zelle orientiert werden und dann in diesem Zustand durch Bestrahlen mit Licht einer geeigneten Wellenlänge vernetzt werden. Die dadurch erzeugte Struktur bleibt auch bei hohen Temperaturen erhalten. So lassen sich optische Bauelemente, wie zum Beispiel Wellenleiter, optische Gitter und Filter, piezoelektrische Zellen und Zellen mit nicht-linearen optischen (NLO) Eigenschaften, usw. herstellen. Solche optische Bauelemente können zum Beispiel für die Frequenzverdoppelung (SHG) oder in Farbfiltern verwendet werden.

Weitere Eigenschaften, wie beispielsweise die Doppelbrechung, der Brechungsindex, die Transparenz, usw., müssen je nach Anwendungsgebiet unterschiedlichen Anforderungen genügen. So sollten Materialien für optische Filter eine starke Absorption in einer Richtung senkrecht zur Filteroberfläche aufweisen.

Neben der generellen Verwendbarkeit von photovernetzbaren Flüssigkristallen für optische Bauelemente, eignen sich solche flüssigkristalline Materialien als Cladding von Glasfibern für die optische Datenübertragung. Der Einsatz solcher Materialien erhöht den elastischen Modulus in der Längsachse der Fiber, verkleinert die thermischen Expansionskoeffizienten und vermindert Mikroverbiegungsverluste. Dies führt zu einer erhöhten mechanischen Stabilität.

Die photovernetzbaren Flüssigkristalle müssen eine gute chemische und thermische Stabilität, gute Löslichkeit in gängigen Lösungsmitteln und eine gute Stabilität gegenüber elektrischen Feldern und elektromagnetischer Strahlung aufweisen. Sie sollten in einem Temperaturbereich von etwa 25°C bis etwa +100°C, insbesondere von etwa 25°C bis etwa +80°C, eine geeignete Mesophase besitzen. Ausserdem ist es wichtig, dass die Komponenten untereinander gut mischbar sind, da Flüssigkristalle in der Regel als Mischungen mehrerer Komponenten zur Anwendung gelangen. Herkömmliche photochemisch oligomerisierbare oder polymerisierbare Flüssigkristalle besitzen in der Regel einen hohen Schmelz- und Klärpunkt. Dies hat den Nachteil, dass vorzeitig eine spontane, thermische Polymerisation eintreten kann bei der Verarbeitung, welche bei Temperaturen knapp unter dem Klärpunkt durchgeführt wird, weil bei dieser Temperatur die Viskosität im flüssigkristallinen Zustand am niedrigsten und daher günstig für eine gute Orientierbarkeit ist. Diese spontane Polymerisation führt zu Domänenbildung, wodurch die optischen und thermischen Eigenschaften in den erzeugten, vernetzten Schichten deutlich beeinträchtigt werden. Der Schmelzpunkt kann durch die Herstellung komplizierter Mischungen mit mehreren Komponenten herabgesetzt werden, was zwar eine Verarbeitung bei niedrigeren Temperaturen erlaubt, aber die Gefahr einer Kristallisation der herkömmlichen polymerisierbaren Flüssigkristalle mit sich bringt. Photochemisch oligomerisierbare oder polymerisierbare Verbindungen werden beispielsweise in EP-A-0 331 233 beschrieben.

Es stellte sich somit die Aufgabe, insbesondere für die Anwendung in optischen Filtern, photochemisch oligomerisierbare oder polymerisierbare Verbindungen herzustellen, welche relativ niedrigere Schmelz- und Klärpunkte besitzen, damit sie bei Temperaturen oberhalb Raumtemperatur im flüssigkristallinen Zustand und auch in Lösung sehr gut verarbeitbar sind. Weiter sollten sie möglichst domänenfrei orientierbar und strukturierbar sein und auch eine ausgezeichnete thermische Stabilität und Langzeitstabilität im vernetzten Zustand aufweisen. Zudem sollten, insbesondere für optische Retarder, Mischungen mit einer einstellbaren optischen Anisotropie zur Verfügung gestellt werden, damit zum Beispiel die optische Verzögerung eines optischen Retarders eingestellt werden kann. Herkömmliche photochemisch oligomerisierbare oder polymerisierbare Flüssigkristalle, wie beispielsweise in EP-A-0 331 233 beschrieben, bestehen aus vorwiegend aromatischen Ringen und weisen daher in der Regel eine sehr grosse optische Anisotropie auf.

Die vorliegende Erfindung stellt nun Verbindungen, die in hervorragender Weise als Einzelkomponenten oder als Komponenten solcher Flüssigkristall-Mischungen geeignet sind, zur Verfügung. Es sind dies Verbindungen der allgemeinen Formel I 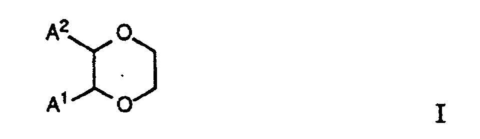 worin
- A¹ und A²: je einen vernetzbaren, mesogenen Rest darstellen.

Da die erfindungsgemässen Verbindungen der Formel I oder Mischungen enthaltend solche Verbindungen eine Mesophase aufweisen, können sie auch vor dem Vernetzen auf einer Orientierungsschicht durch Anlegen eines elektrischen oder magnetischen Feldes orientiert werden. Dabei wird eine einheitliche Schicht erzeugt.

Die mesogenen Reste A¹ und A² bedeuten je einen Rest der allgemeinen Formel II 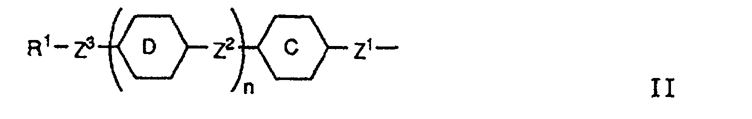 worin
- Ringe C und D: unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
- Z¹: -CH₂-(CH₂)ₘ-, -(CH₂)ₘO-, -O(CH₂)ₘ-, -COO-, -OOC-, -(CH₂)ₘCOO- oder -(CH₂)ₘOOC-;
- Z²: eine Einfachbindung, -CH₂CH₂-, -CH₂O-, -OCH₂-, -COO-, -OOC-, -(CH₂)₄-, -O(CH₂)₃- oder -(CH₂)₃O-;
- Z³: -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, oder -NHCH₂(Si[(CH₃)₂]O)ₘSi[(CH₃)₂]CH₂NH-;
- Y: Wasserstoff oder Fluor;
- n: 0, 1 oder 2;
- m: eine ganze Zahl von 1 bis 16; und
- R¹: vernetzbare Gruppen der Struktur CH2=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 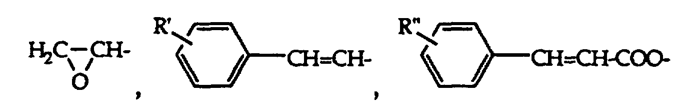
- Ph: Phenyl;
- R': niederes Alkyl;
- R": Methyl, Methoxy, Cyano oder Halogen bedeuten;
mit der Massgabe, dass R¹-Z³ keine -O-O- oder -N-O- Gruppen enthalten.

Bevorzugt werden Verbindungen der Formel I, worin die beiden mesogenen Reste A¹ und A² die gleiche Bedeutung haben.

Besonders bevorzugt werden Reste A¹ und A² der Formel II, worin die Ringe C und D unabhängig voneinander Pyridin-2,5-diyl, Pyrimidin-2,5-diyl, unsubstituiertes oder mit Fluor substituiertes 1,4-Phenylen; Z¹ -CH₂CH₂-, -CH₂O-, -COO- oder -OOC- ; Z² eine Einfachbindung, -CH₂CH₂-, -CH₂O-, -OCH₂-, -COO- oder -OOC-; Z³ -(CH₂)ₘ-, -(CH₂)ₘO-, -(CH₂)ₘCOO- oder -(CH₂)ₘOOC- bedeuten.

Vorzugsweise bedeutet die vernetzbare Gruppe R¹ CH₂=CH-COO-, CH₂=C(CH₃)-COO-, CH₂=C(Cl)-COO-, CH₂=C(Ph)-COO-, CH₂=CH-COO-Ph-CH₂=CH-CONH-, CH₂=C(CH₃)-CONH-, CH₂=C(Ph)-CONH-, CH₂=CH-O-, CH₂=CH-OOC-, -cis, trans -HOO-CR'=CR'-COO-, 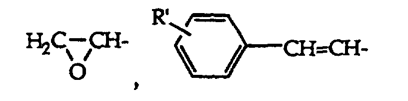 worin R' und R" die oben angegebene Bedeutung haben.

Es sind dies Reste, welche nach Orientieren der Verbindungen der Formel I in einem Feld photochemisch vernetzt werden können.

Besonders bevorzugte Gruppen R¹ sind CH₂=CH-COO-, CH₂=C(CH₃)-COO-, CH₂=CH-O-,

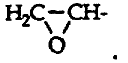

Die oben verwendeten Ausdrücke werden im Folgenden erläutert:

"Gegebenenfalls mit Halogen, Methyl und/oder Cyano substituiertes 1,4-Phenylen" umfasst in der vorliegenden Erfindung 1,4-Phenylen, mit Fluor, Brom, Chlor, Methyl oder Cyano einfach oder mehrfach substituiertes 1,4-Phenylen wie beispielsweise 2- bzw. 3-Fluor-1,4-phenylen, 2,3-Difluor-1,4-phenylen, 2,6- bzw. 3,5-Difluor-1,4-phenylen, 2- bzw. 3-Chlor-1,4-phenylen, 2,3-Dichlor-1,4-phenylen, 2,6- bzw. 3,5-Dichlor-1,4-phenylen, 2-bzw. 3-Brom-1,4-phenylen, 2- bzw. 3-Methyl-1,4-phenylen, 2- bzw. 3-Cyano-1,4-phenylen und dergleichen.

" Niederes Alkyl" umfasst im Rahmen der vorliegenden Erfindung geradkettige oder verzweigte Reste mit 1 bis 5 Kohlenstoffatomen, wie Methyl, Ethyl, Propyl, Butyl, Pentyl, i-Propyl, i-Butyl, t. Butyl und dergleichen.

"Halogen" steht für Fluor, Chlor oder Brom, insbesondere für Fluor.

"Der Mesophasen-Typ der erfindungsgemässen Verbindungen kann durch Variation der Ringe in den Seitenketten A¹ und A² beeinflusst werden. So haben aromatische Ringe wie Phenylen die Tendenz smektische Phasen zu erzeugen, während gesättigte Ringe wie trans-1,4-Cyclohexylen oder trans-1,3-Dioxan-2,5-diyl Ringe nematische Tendenzen fördern.

Vorzugsweise bedeuten die mesogenen Reste A¹ und A² einen Rest der Formel II, worin n = 1 ist, das heisst einen Rest der Formel II-a 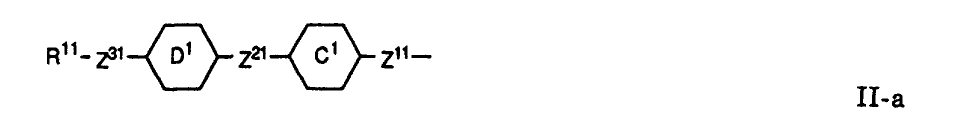 worin
- Ringe C¹ und D¹: Pyridin-2,5-diyl, Pyrimidin-2,5-diyl oder ein gegebenenfalls mit Fluor substituiertes 1,4-Phenylen;
- Z¹¹: -CH₂CH₂-, -CH₂O-, -COO- oder -OOC-;
- Z²¹: eine Einfachbindung,-CH₂O-, -COO-, -OOC-;
- Z³¹: -(CH₂)_{m'}-, -(CH₂)_{m'}O-, -(CH₂)_{m'}COO- oder -(CH₂)_{m'}OOC-;
- m': eine ganze Zahl von 3 bis 12; und
- R¹¹: CH₂=CH-COO-, CH₂=C(CH₃)-COO-, CH₂=CH-O-, 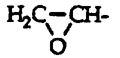
bedeuten.

Ganz besonders bevorzugt sind Verbindungen der Formeln 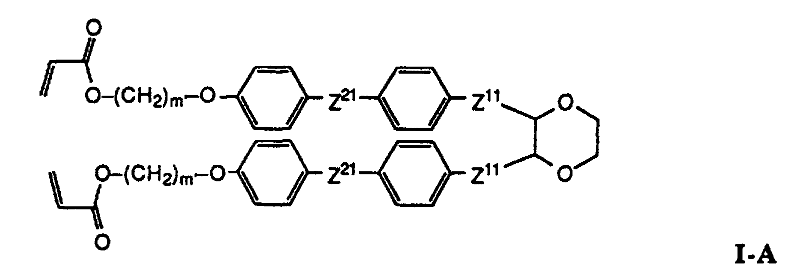 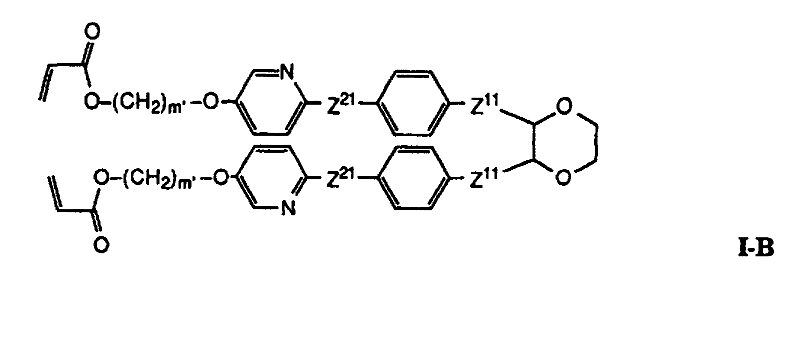 und 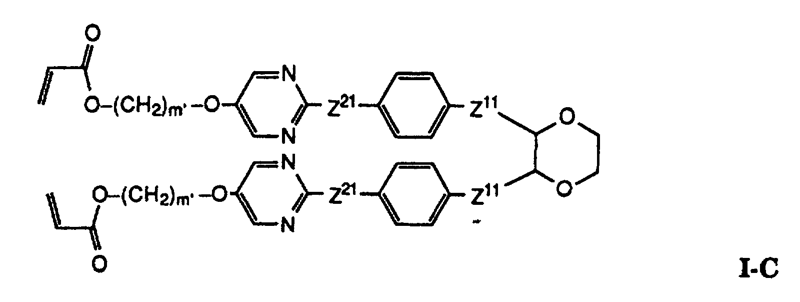 worin
- Z¹¹: -COO-;
- Z²¹: eine Einfachbindung oder -COO-, insbesondere -COO-;
- m': eine ganze Zahl von 3 bis 12 bedeuten.

Die Verbindungen der Formel I, worin A¹ und A² die gleiche Bedeutung haben, sind synthetisch sehr einfach zugänglich und können beispielsweise analog zu den in den Schemata 1 bis 5 aufgezeigten Methoden hergestellt werden. So kann in an sich bekannter Weise trans-2,3-Dihydroxy-1,4-dioxan mit (ω-acryloyloxyalkyloxy)-substituierten Carbonsäuren umgesetzt werden. Diese Veresterung kann beispielsweise über das entsprechende Methylsulfonat in Tetrahydrofuran oder in Gegenwart von N,N'-Dicyclohexylcarbodiimid und 4-(Dimethylamino)pyridin in Dichlormethan oder einem anderen geeigneten Lösungsmittel wie z.B. Chloroform erfolgen. trans-2,3-Dihydroxy-1,4-dioxan kann auch in einer Williamson Veretherung mit (ω-acryloyloxyalkyloxy)-substituierten Benzyltosylaten umgesetzt werden. Diese Veretherung kann beispielsweise bei Raumtemperatur in Gegenwart von Kalium-tert.-butylat in Dimethoxyethan oder einem anderen geeigneten Lösungsmittel, wie z.B. N, N'-Dimethylfomamid, erfolgen. trans-2,3-Dihydroxy-1,4-dioxan ist kommerziell erhältlich.

Verbindungen der Formel I, worin A¹ und A² verschieden sind, können durch Monoveresterung von trans-2,3-Dihydroxy-1,4-dioxan mit einer (ω-acryloyloxyalkyloxy)-substituierten Carbonsäure hergestellt werden. Anschliessende Veresterung mit einer anderen (ω-acryloyloxyalkyloxy)-substituierten Carbonsäure ergibt den asymmetrischen Diester. Die entsprechenden asymmetrischen Diether sind über ein analoges zweistufiges Verfahren zugänglich, in welchem beispielsweise trans-2,3-Dihydroxy-1,4-dioxan zunächst mit einem (ω-acryloyloxyalkyloxy)-substituierten Benzyltosylat monoalkyliert und anschliessend mit einem anderen (ω-acryloyloxyalkyloxy)-substituierten Benzyltosylat alkyliert wird. Die Ausgangsmaterialien sind bekannt und z.T. im Handel erhältlich.

Die in den Schemata 1 bis 5 aufgezeichneten Reaktionen sind Standardmethoden in der Flüssigkristall-Chemie. Die darin verwendeten Symbole haben die obengenannten Bedeutungen.

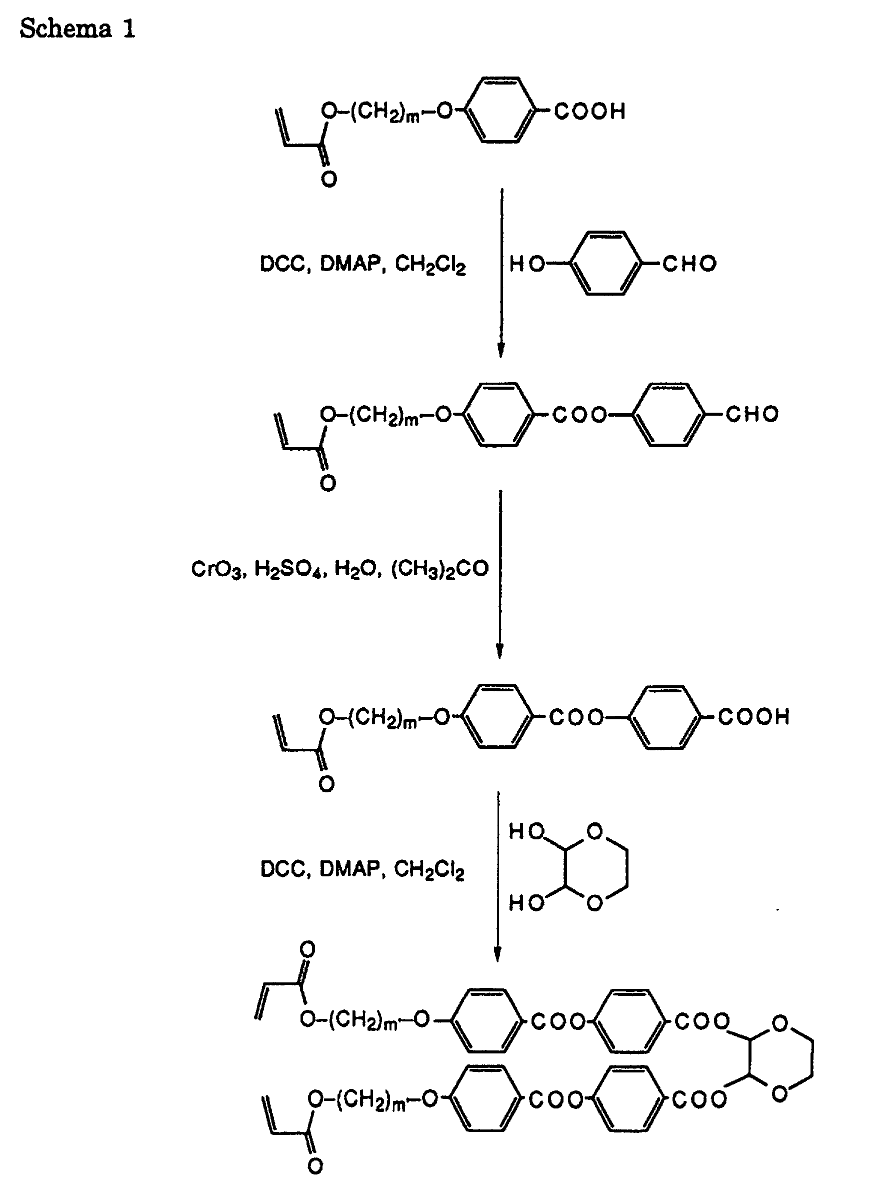

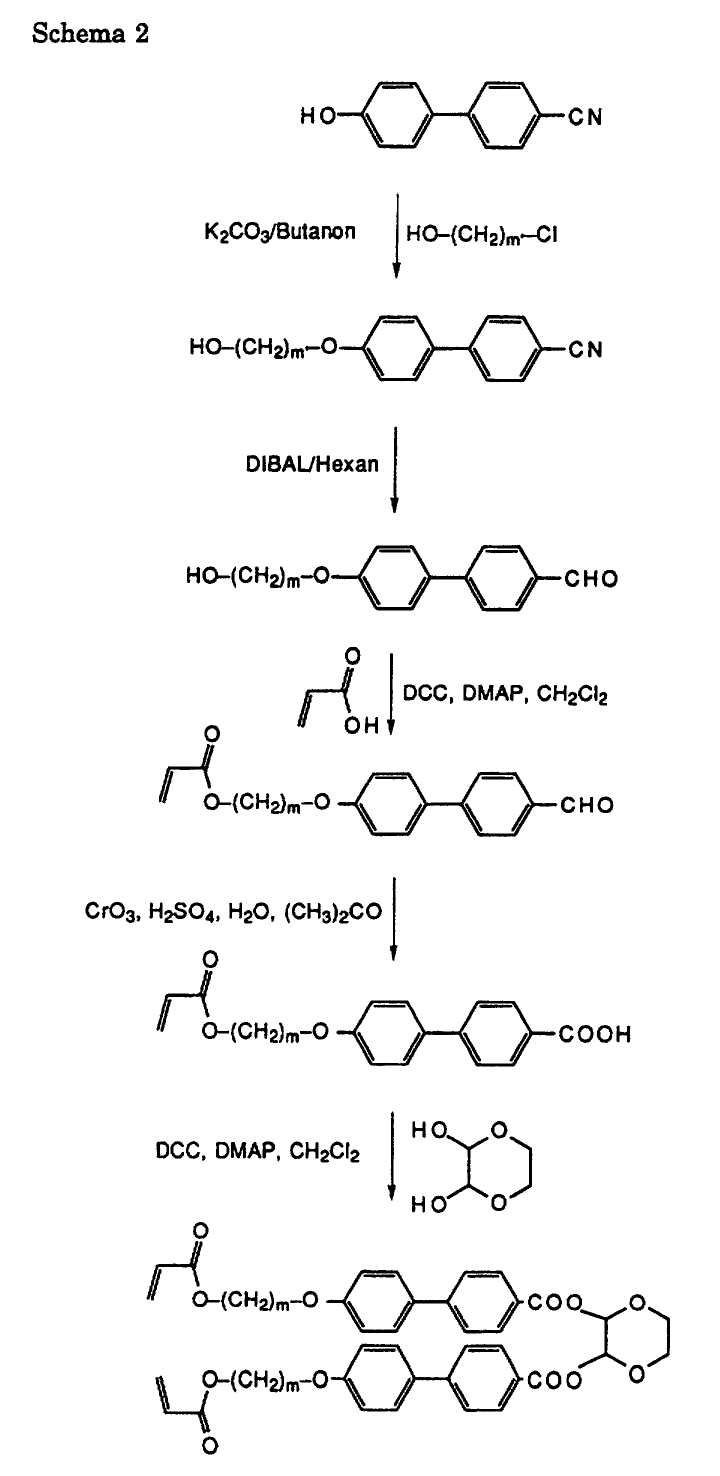

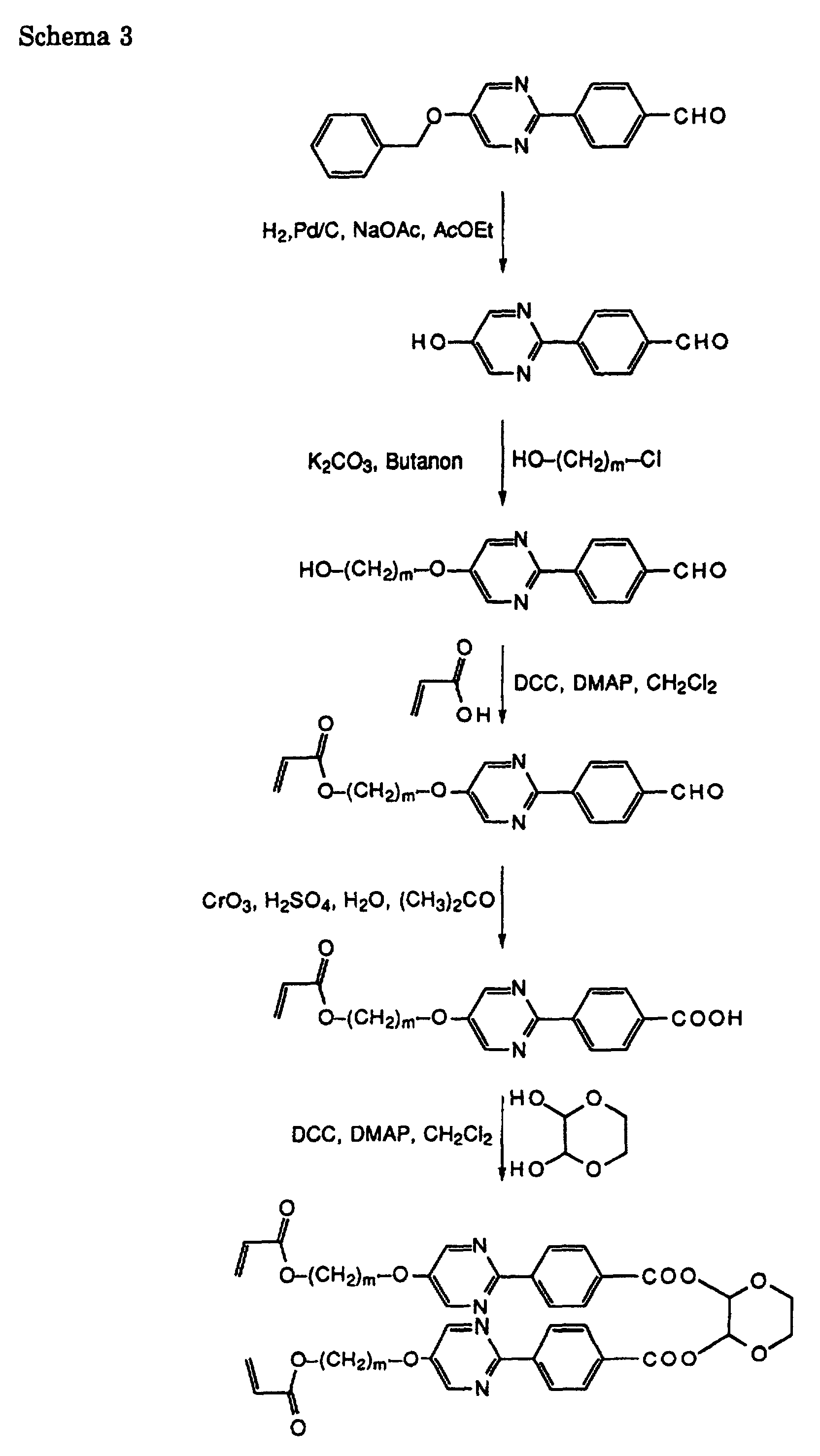

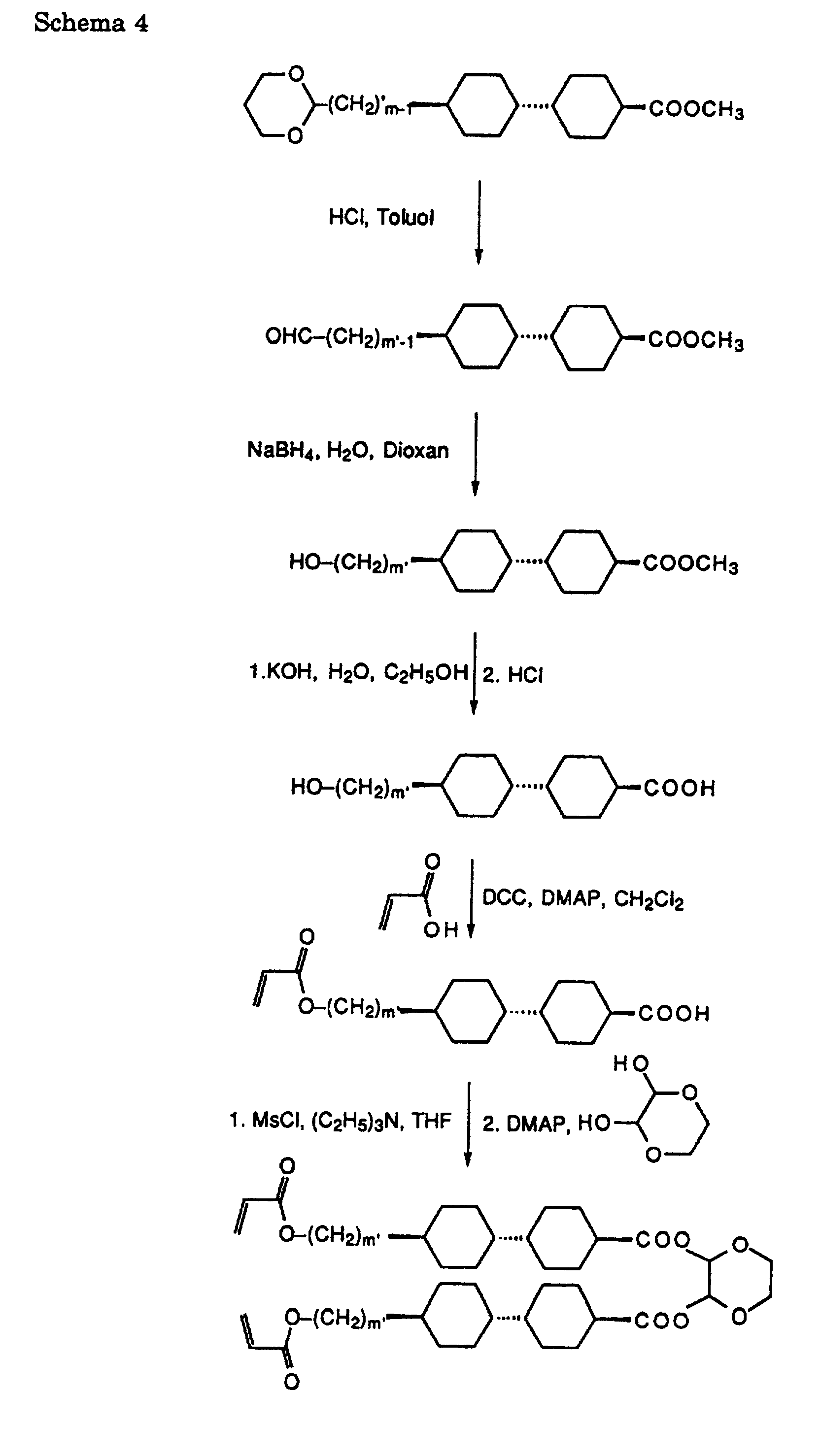

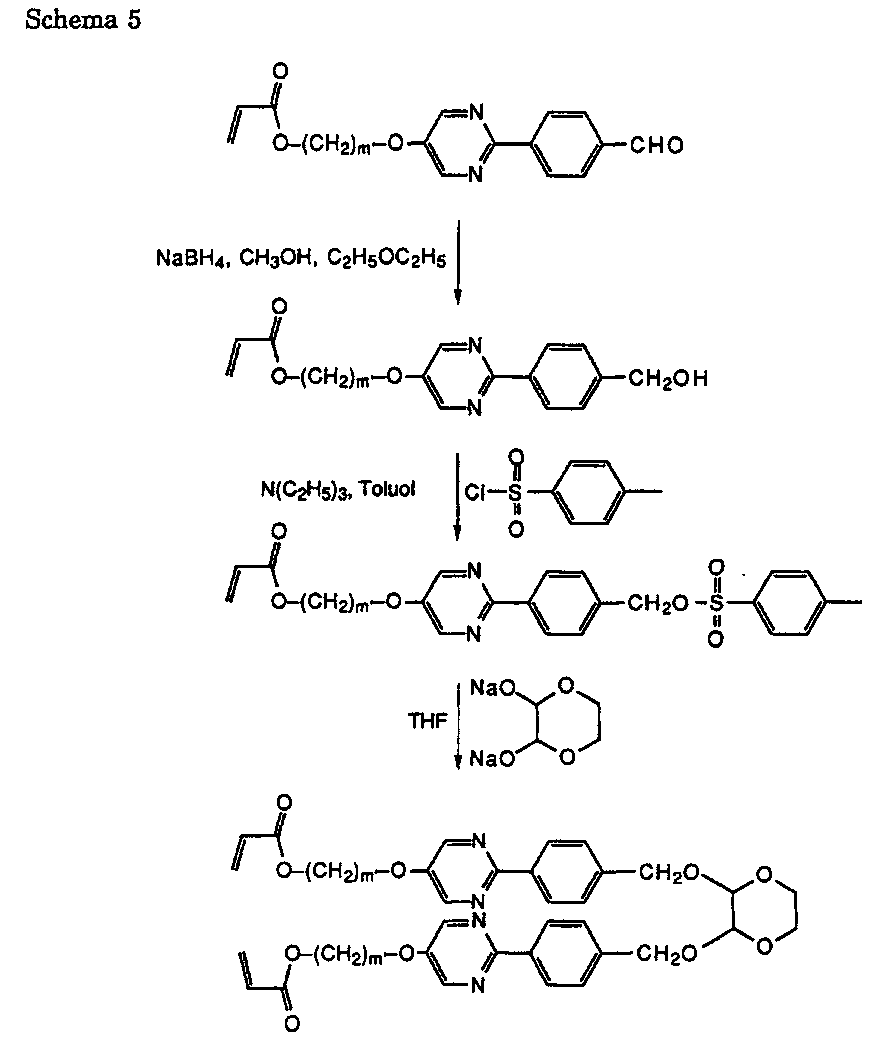

Eine kleine Menge BHT (2,6-Di-tert.-butyl-4-methyl-phenol/"Butylhydroxytoluol") wird jeder Stufe beigemischt, um unerwünschtes thermisches Vernetzen zu unterbinden.

Die Verbindungen der Formeln I können als reine Verbindungen oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder andere, bekannte flüssigkristalline Verbindungen mit einer photovernetzbaren Gruppe sein. Es können auch eine oder mehrere chirale Komponenten im Gemisch enthalten sein.

Aufgrund der guten Löslichkeit der Verbindungen der Formel I und aufgrund ihrer guten Mischbarkeit untereinander kann der Anteil an Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 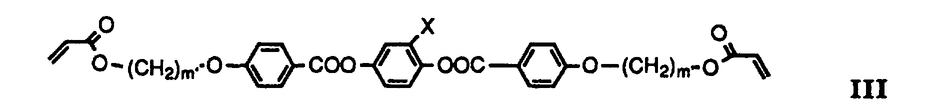 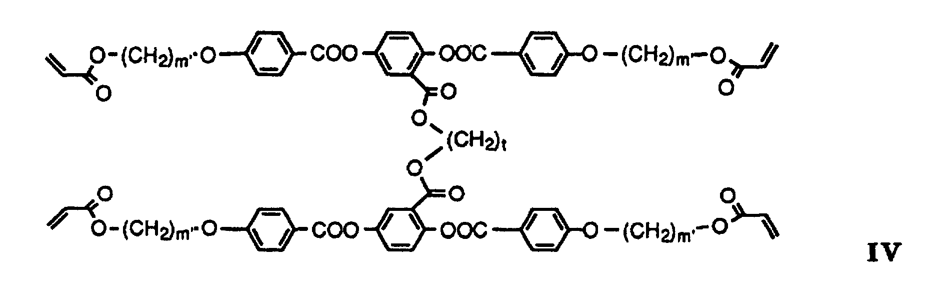 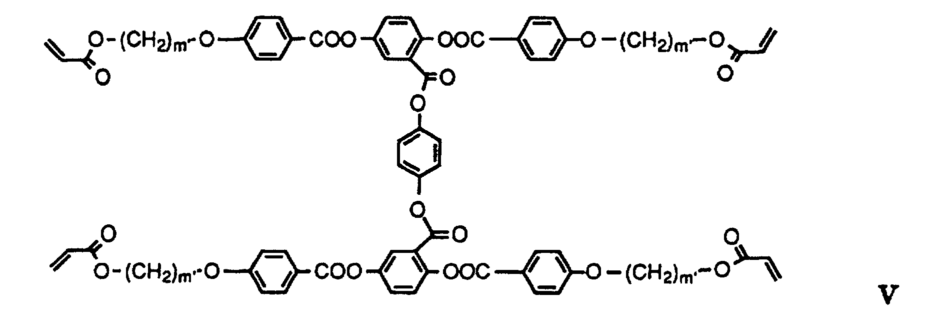 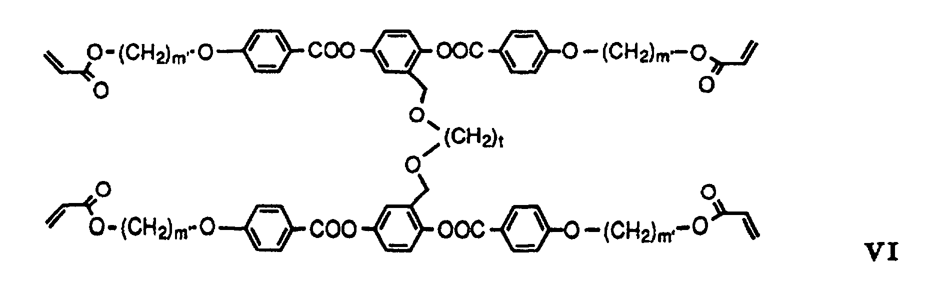 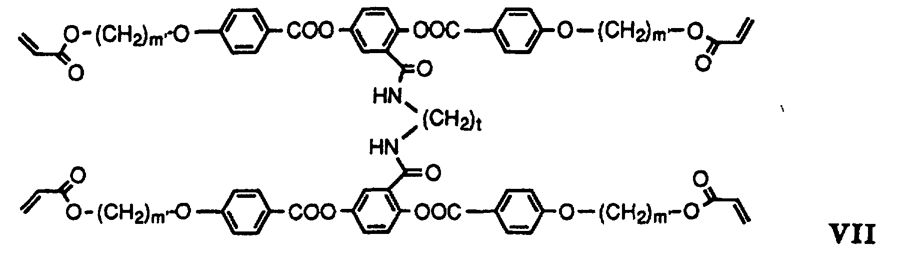 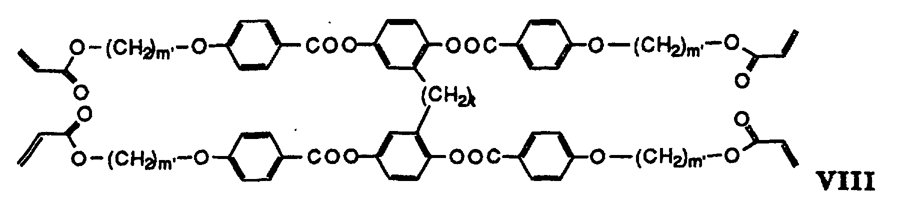 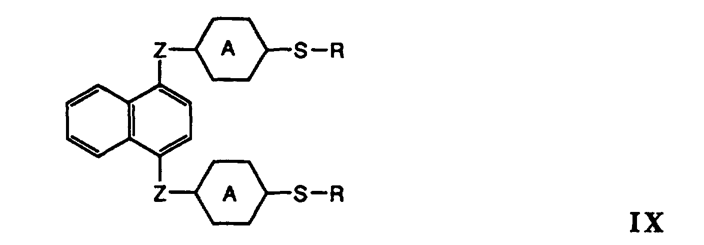 und 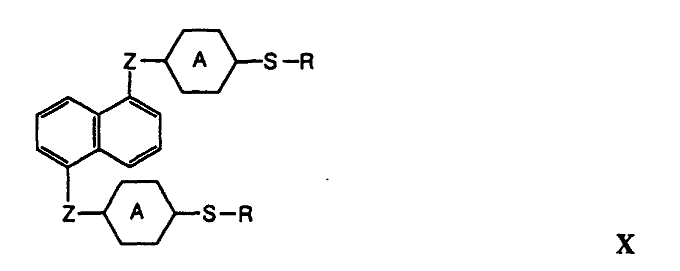 worin
- X: Wasserstoff, Fluor, Chlor, Brom oder Methyl;
- m': eine ganze Zahl von 3 bis 12;
- t: eine ganze Zahl von 2 bis 12;
- Z: -OCH₂- oder -OOC-;
- A: 1,4-Phenylen oder 2- bzw. 3-Fluor-1,4-phenylen;
- S: -(CH₂)_{m'} - oder -(CH₂)_{m'}O-; und
- R: CH₂=CH-COO-, CH₂=C(CH₃)-COO-, CH₂=CH-O-, 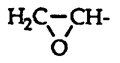
bedeuten.

Die Herstellung der Verbindungen der Formel I sowie flüssigkristalline Gemische enthaltend diese Verbindungen werden durch die folgenden Beispiele weiter veranschaulicht. C bedeutet eine kristalline, S eine smektische, N eine nematische und I die isotrope Phase.

### Beispiel 1

Zu einer Lösung von 0,1 g trans-2,3-Dihydroxy-1,4-dioxan, 0,9 g 4-(4-[8-Acryloyloxyoctyloxy]phenylcarbonyloxy)benzoesäure und 0,05 g 4-(Dimethylamino)pyridin in 25 ml Dichlormethan wurde unter Rühren innert 5 Minuten 0,43 g N,N '-Dicyclohexylcarbodiimid gegeben. Das Reaktionsgemisch wurde über Nacht gerührt, filtriert und das Filtrat eingeengt. Chromatographie des Rückstandes an Kieselgel mit Hexan/Ethylacetat (Vol. 8:2) und Umkristallisation der gemäss Dünnschichtchromatographie reinen Fraktionen aus Ethylalkohol ergab 0,15 g trans-2,3-bis[4-(4-[8-Acryloyloxyoctyloxy]phenylcarbonyloxy)phenylcarbonyloxy)-1,4-dioxan; Smp. (C-N) 98°C und Klp. (N-I) 153°C.

In analoger Weise können folgende Verbindungen hergestellt werden:
trans-2,3-bis[4-(4-[3-Acryloyloxypropyloxy]phenylcarbonyloxy)phenylcarbonyloxy]-1,4-dioxan;
trans-2,3-bis[4-(4-[4-Acryloyloxybutoxy]phenylcarbonyloxy)phenylcarbonyloxy]-1,4-dioxan;
trans-2,3-bis[4-(4-[5-Acryloyloxypentyloxy]phenylcarbonyloxy)phenylcarbonyloxy]-1,4-dioxan;
trans-2,3-bis[4-(4-[6-Acryloyloxyhexyloxy]phenylcarbonyloxy)phenylcarbonyloxy]-1,4-dioxan;
trans-2,3-bis[4-(4-[7-Acryloyloxyheptyloxy]phenylcarbonyloxy)phenylcarbonyloxy]-1,4-dioxan;
trans-2,3-bis[4-(4-[9-Acryloyloxynonyloxy]phenylcarbonyloxy)phenylcarbonyloxy]-1,4-dioxan;
trans-2,3-bis[4-(4-[10-Acryloyloxydecyloxy]phenylcarbonyloxy)phenylcarbonyloxy]-1,4-dioxan;
trans-2,3-bis[4-(4-[11-Acryloyloxyundecyloxy]phenylcarbonyloxy)phenylcarbonyloxy]-1,4-dioxan;
trans-2,3-bis[4-(4-[12-Acryloyloxydodecyloxy]phenylcarbonyloxy)phenylcarbonyloxy]-1,4-dioxan;
trans-2,3-bis(4-[3-Acryloyloxypropyloxy]biphenyl-4'-carbonyloxy)-1,4-dioxan;
trans-2,3-bis(4-[4-Acryloyloxybutoxy]biphenyl-4'-carbonyloxy)-1,4-dioxan;
trans-2,3-bis(4-[5-Acryloyloxypentyloxy]biphenyl-4'-carbonyloxy)-1,4-dioxan;
trans-2,3-bis(4-[6-Acryloyloxyhexyloxy]biphenyl-4'-carbonyloxy)-1,4-dioxan;
trans-2,3-bis(4-[7-Acryloyloxyheptyloxy]biphenyl-4'-carbonyloxy)-1,4-dioxan;
trans-2,3-bis(4-[8-Acryloyloxyoctyloxy]biphenyl-4'-carbonyloxy)-1,4-dioxan;
trans-2,3-bis(4-[9-AcryloyloxynonyloxyJbiphenyl-4'-carbonyloxy)-1,4-dioxan;
trans-2,3-bis(4-[10-Acryloyloxydecyloxy]biphenyl-4'-carbonyloxy)-1,4-dioxan;
trans-2,3-bis(4-[11-Acryloyloxyundecyloxy]biphenyl-4'-carbonyloxy)-1,4-dioxan;
trans-2,3-bis(4-[12-Acryloyloxydodecyloxy]biphenyl-4'-carbonyloxy)-1,4-dioxan;
trans-2,3-bis[4-(5-[3-Acryloyloxypropyloxy]pyrimidin-2-yl)phenylcarbonyloxy]-1,4-dioxan;
trans-2,3-bis[4-(5-[4-Acryloyloxybutyloxy]pyrimidin-2-yl)phenylcarbonyloxy]-1,4-dioxan;
trans-2,3-bis[4-(5-[5-Acryloyloxypentyloxy]pyrimidin-2-yl)phenylcarbonyloxy]-1,4-dioxan;
trans-2,3-bis[4-(5-[6-Acryloyloxyhexyloxy]pyrimidin-2-yl)phenylcarbonyloxy]-1,4-dioxan;
trans-2,3-bis[4-(5-[7-Acryloyloxyheptyloxy)pyrimidin-2-yl)phenylcarbonyloxy]-1,4-dioxan;
trans-2,3-bis[4-(5-[8-Acryloyloxyoctyloxy]pyrimidin-2-yl)phenylcarbonyloxy]-1,4-dioxan;
trans-2,3-bis[4-(5-[9-Acryloyloxynonyloxy)pyrimidin-2-yl)phenylcarbonyloxy]-1,4-dioxan;
trans-2,3-bis[4-(5-[10-Acryloyloxydecyloxy]pyrimidin-2-yl)phenylcarbonyloxy]-1,4-dioxan;
trans-2,3-bis[4-(5-[11-Acryloyloxyundecyloxy]pyrimidin-2-yl)phenylcarbonyloxy]-1,4-dioxan;
trans-2,3-bis[4-(5-[12-Acryloyloxydodecyloxy]pyrimidin-2-yl)phenylcarbonyloxy]-1,4-dioxan;
trans-2,3-bis[4-(5-[3-Acryloyloxypropyloxy]pyridin-2-yl)phenylcarbonyloxy]-1,4-dioxan;
trans-2,3-bis[4-(5-[4-Acryloyloxybutyloxy]pyridin-2-yl)phenylcarbonyloxy]-1,4-dioxan;
trans-2,3-bis[4-(5-[5-Acryloyloxypentyloxy]pyridin-2-yl)phenylcarbonyloxy]-1,4-dioxan;
trans-2,3-bis[4-(5-[6-Acryloyloxyhexyloxy]pyridin-2-yl)phenylcarbonyloxy]-1,4-dioxan;
trans-2,3-bis[4-(5-[7-Acryloyloxyheptyloxy]pyridin-2-yl)phenylcarbonyloxy]-1,4-dioxan;
trans-2,3-bis[4-(5-[8-Acryloyloxyoctyloxy]pyridin-2-yl)phenylcarbonyloxy]-1,4-dioxan;
trans-2,3-bis[4-(5-[9-Acryloyloxynonyloxy]pyridin-2-yl)phenylcarbonyloxy]-1,4-dioxan;
trans-2,3-bis[4-(5-[10-Acryloyloxydecyloxy]pyridin-2-yl)phenylcarbonyloxy]-1,4-dioxan;
trans-2,3-bis[4-(5-[11-Acryloyloxyundecyloxy]pyridi-2-yl)phenylcarbonyloxy]-1,4-dioxan;
trans-2,3-bis[4-(5-[12-Acryloyloxydodecyloxy]pyridin-2-yl)phenylcarbonyloxy]-1,4-dioxan;
trans-2,3-bis[2-(4-[8-Acryloyloxyoctyloxy]phenyl)pyrimidin-5-ylcarbonyloxy]-1,4-dioxan;
trans-2,3-bis[2-(4-[8-Acryloyloxyoctyloxy]phenyl)pyridin-5-ylcarbonyloxy]-1,4-dioxan;
trans-2,3-bis[trans-4-(trans-4-[3-Acryloyloxypropyl]cyclohexyl)cyclohexancarbonyloxy]-1,4-dioxan;
trans-2,3-bis[trans-4-(2-[trans-4-(3-Acryloyloxypropyl)cyclohexyl]ethyl)cyclohexancarbonyloxy]-1,4-dioxan;
trans-2,3-bis[4-(trans-4-[3-acryloyloxypropyl]cyclohexyl)phenylcarbonyloxy]-1,4-dioxan;
trans-2,3-bis[4-(2-[trans-4-(3-Acryloyloxypropyl)cyclohexyl]ethyl)phenylcarbonyloxy]-1,4-dioxan.

### Beispiel 2

Eine Lösung aus 0,2 g trans-2,3-Dihydroxy-1,4-dioxan, 0,5 g Natriumhydrid und 50 ml Tetrahydrofuran wird 7 Stunden gerührt. 1,1 g 4-(5-[8-Acryloyloxyoctyloxy]pyrimidin-2-yl)benzyltosylat wird zugegeben und das Gemisch über Nacht bei Raumtemperatur gerührt, auf 100 ml Wasser gegossen und dann dreimal mit je 50 ml Diethylether extrahiert. Die organischen Phasen werden vereinigt, zweimal mit je 100 ml Wasser gewaschen, über Magnesiumsulfat getrocknet, filtriert und das Filtrat eingeengt. Chromatographische Reinigung des Rückstandes an Kieselgel mit Cyclohexan/Ethylacetat (Vol. 8:2) und zweimaliges Umkristallisieren der gemäss Dünnschichtchromatographie reinen Fraktionen aus einem Cyclohexan/Ethylacetat Gemisch (Vol. 8:2) ergibt 1,1 g trans-2,3-bis[4-(5-[8-Acryloyloxyoctyloxy]pyrimidin-2-yl)benzyloxy]-1,4-dioxan.

In analoger Weise können folgende Verbindungen hergestellt werden:
trans-2,3-bis[4-(4-[3-Acryloyloxypropyloxy]phenylcarbonyloxy)benzyloxy]-1,4-dioxan;
trans-2,3-bis[4-(4-[4-Acryloyloxybutoxy]phenylcarbonyloxy)benzyloxy]-1,4-dioxan;
trans-2,3-bis[4-(4-[5-Acryloyloxypentyloxy]phenylcarbonyloxy)benzyloxy]-1,4-dioxan;
trans-2,3-bis[4-(4-[6-Acryloyloxyhexyloxy]phenylcarbonyloxy)benzyloxy]-1,4-dioxan;
trans-2,3-bis[4-(4-[7-Acryloyloxyheptyloxy]phenylcarbonyloxy)benzyloxy]-1,4-dioxan;
trans-2,3-bis[4-(4-[9-Acryloyloxynonyloxy]phenylcarbonyloxy)benzyloxy]-1,4-dioxan;
trans-2,3-bis[4-(4-[10-Acryloyloxydecyloxy]phenylcarbonyloxy)benzyloxy]-1,4-dioxan;
trans-2,3-bis[4-(4-[11-Acryloyloxyundecyloxy]phenylcarbonyloxy)benzyloxy]-1,4-dioxan;
trans-2,3-bis[4-(4-[12-Acryloyloxydodecyloxy]phenylcarbonyloxy)benzyloxy]-1,4-dioxan;
trans-2,3-bis(4-[3-Acryloyloxypropyloxy]biphenyl-4'-methoxy)-1,4-dioxan;
trans-2,3-bis(4-[4-Acryloyloxybutoxy]biphenyl-4'-methoxy)-1,4-dioxan;
trans-2,3-bis(4-[5-Acryloyloxypentyloxy]biphenyl-4'-methoxy)-1,4-dioxan;
trans-2,3-bis(4-[6-Acryloyloxyhexyloxy]biphenyl-4'-methoxy)-1,4-dioxan;
trans-2,3-bis(4-[7-Acryloyloxyheptyloxy]biphenyl-4'-methoxy)-1,4-dioxan;
trans-2,3-bis(4-[8-Acryloyloxyoctyloxy]biphenyl-4'-methoxy)-1,4-dioxan;
trans-2,3-bis(4-[9-Acryloyloxynonyloxy]biphenyl-4'-methoxy)-1,4-dioxan;
trans-2,3-bis(4-[10-Acryloyloxydecyloxy]biphenyl-4'-methoxy)-1,4-dioxan;
trans-2,3-bis(4-[11-Acryloyloxyundecyloxy]biphenyl-4'-methoxy)-1,4-dioxan;
trans-2,3-bis(4-[12-Acryloyloxydodecyloxy]biphenyl-4'-methoxy)-1,4-dioxan;
trans-2,3-bis[4-(5-[3-Acryloyloxypropyloxy]pyrimidin-2-yl)benzyloxy]-1,4-dioxan;
trans-2,3-bis[4-(5-[4-Acryloyloxybutyloxy]pyrimidin-2-yl)benzyloxy]-1,4-dioxan;
trans-2,3-bis[4-(5-[5-Acryloyloxypentyloxy]pyrimidin-2-yl)benzyloxy]-1,4-dioxan;
trans-2,3-bis[4-(5-[6-Acryloyloxyhexyloxy]pyrimidin-2-yl)benzyloxy]-1,4-dioxan;
trans-2,3-bis[4-(5-[7-Acryloyloxyheptyloxy]pyrimidin-2-yl)benzyloxy]-1,4-dioxan;
trans-2,3-bis[4-(5-[8-Acryloyloxyoctyloxy]pyrimidin-2-yl)benzyloxy]-1,4-dioxan;
trans-2,3-bis[4-(5-[9-Acryloyloxynonyloxy]pyrimidin-2-yl)benzyloxy]-1,4-dioxan;
trans-2,3-bis[4-(5-[10-Acryloyloxydecyloxy]pyrimidin-2-yl)benzyloxy]-1,4-dioxan;
trans-2,3-bis[4-(5-[11-Acryloyloxyundecyloxy]pyrimidin-2-yl)benzyloxy]-1,4-dioxan;
trans-2,3-bis[4-(5-[12-Aclyloyloxydodecyloxy]pyrimidin-2-yl)benzyloxy]-1,4-dioxan;
trans-2,3-bis[4-(5-[3-Acryloyloxypropyloxy]pyridin-2-yl)benzyloxy]-1,4-dioxan;
trans-2,3-bis[4-(5-[4-Acryloyloxybutyloxy]pyridin-2-yl)benzyloxy]-1,4-dioxan;
trans-2,3-bis[4-(5-[5-Acryloyloxypentyloxy]pyridin-2-yl)benzyloxy]-1,4-dioxan;
trans-2,3-bis[4-(5-[6-Acryloyloxyhexyloxy]pyridin-2-yl)benzyloxy]-1,4-dioxan;
trans-2,3-bis[4-(5-[7-Acryloyloxyheptyloxy]pyridin-2-yl)benzyloxy]-1,4-dioxan;
trans-2,3-bis[4-(5-[8-Acryloyloxyoctyloxy]pyridin-2-yl)benzyloxy]-1,4-dioxan;
trans-2,3-bis[4-(5-[9-Acryloyloxynonyloxy]pyridin-2-yl)benzyloxy]-1,4-dioxan;
trans-2,3-bis[4-(5-[10-Acryloyloxydecyloxy]pyridin-2-yl)benzyloxy]-1,4-dioxan;
trans-2,3-bis[4-(5-[11-Acryloyloxyundecyloxy]pyridin-2-yl)benzyloxy]-1,4-dioxan;
trans-2,3-bis[4-(5-[12-Acryloyloxydodecyloxy]pyridin-2-yl)benzyloxy]-1,4-dioxan;
trans-2,3-bis[4-(5-[8-Acryloyloxyoctyloxy]pyridin-2-yl)benzyloxy]-1,4-dioxan;
trans-2,3-bis[(2-[4-(8-Acryloyloxyoctyloxy)phenyl]pyrimidin-5-yl)methoxy]-1,4-dioxan;
trans-2,3-bis[(trans-4-(trans-4-[3-Acryloyloxypropyl]cyclohexyl)cyclohexyl)methoxy]-1,4-dioxan;
trans-2,3-bis[(trans-4-[2-(trans-4-[3-Acryloyloxypropyl]cyclohexyl)-ethyl]cyclohexyl)methoxy]-1,4-dioxan;
trans-2,3-bis[4-(trans-4-[3-acryloyloxypropyl]cyclohexyl)benzyloxy]-1,4-dioxan;
trans-2,3-bis[4-(2-[trans-4-(3-Acryloyloxypropyl)cyclohexyl]ethyl)-benzyloxy]-1,4-dioxan.

## Patentansprüche

1. Verbindungen der allgemeinen Formel I 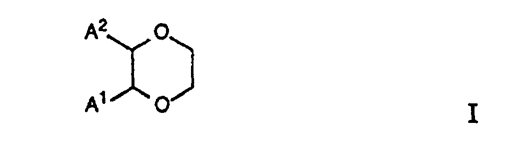 worin
A¹ und A² mesogene Reste der allgemeinen Formel II 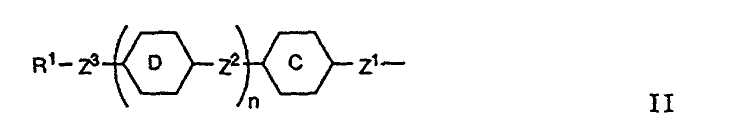 bedeuten, worin
Ringe C und D unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
Z¹ -CH₂-(CH₂)ₘ-, -(CH₂)ₘO-, -O(CH₂)ₘ-, -COO-, -OOC-, -(CH₂)ₘCOO- oder -(CH2)ₘOOC-;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, oder -NHCH₂(Si[(CH₃)₂]O)ₘSi[(CH₃)₂]CH₂NH-;
Y Wasserstoff oder Fluor;
n 0, 1 oder 2;
m eine ganze Zahl von 1 bis 16; und
R¹ vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 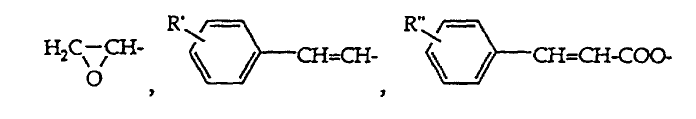
Ph Phenyl;
R' niederes Alkyl;
R" Methyl, Methoxy, Cyano oder Halogen bedeuten;
mit der Massgabe, dass R¹-Z³ keine -O-O- oder -N-O- Gruppen enthalten.

2. Verbindungen nach Anspruch 1, worin worin die mesogenen Reste A¹ und A² Reste der allgemeinen Formel II 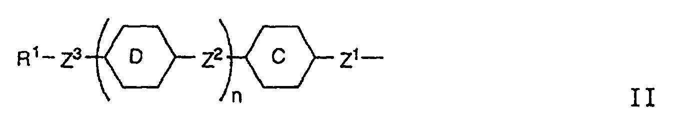 bedeuten, worin
Ringe C und D unabhängig voneinander Pyridin-2,5-diyl, Pyrimidin-2,5-diyl, trans-1,4-Cyclohexylen, trans-1,3-Dioxan-2,5-diyl oder gegebenenfalls mit Halogen, Methyl und/oder Cyano substituiertes 1,4-Phenylen;
Z¹ -CH₂-CH₂-, -CH₂O-, -COO-, -OOC-, -(CH₂)₄- oder -(CH₂)₃O-;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, oder -NHCH₂(Si[(CH₃)₂]O)ₘSi[(CH₃)₂]CH₂NH-;
Y Wasserstoff oder Fluor;
n 0, oder 1;
m eine ganze Zahl von 1 bis 16; und
R¹ vernetzbare Gruppen der Struktur CH₂=CH-, CH₂=CH-COO-, CH₂=C(CH₃)-COO-, CH₂=C(Cl)-COO-, CH₂=C(Ph)-COO-, CH₂=CH-COO-Ph-, CH₂=CH-CO-NH-, CH₂=C(CH₃)-CONH-, CH₂=C(Cl)-CONH-, CH₂=C(Ph)-CONH-, CH₂=C(COOR')-CH₂-COO-, CH₂=CH-O-, CH₂=CH-OOC-, Ph-CH=CH-, CH₃-C(=NR')-, -cis,-trans HOO-CR'=CR'-COO-, 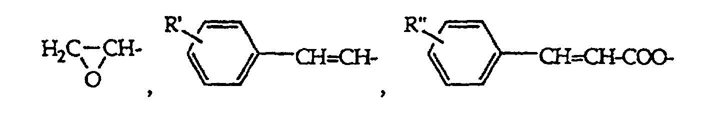
Ph Phenyl;
R' niederes Alkyl;
R" Methyl, Methoxy, Cyano oder Halogen bedeuten;
mit der Massgabe, dass R¹-Z³ keine -O-O- oder -N-O- Gruppen enthalten.

3. Verbindungen nach Anspruch 1 oder 2, worin die mesogenen Reste A¹ und A2 die gleiche Bedeutung haben.

4. Verbindungen nach Anspruch 3, worin die Reste A¹ und A² einen Rest der Formel II-a bedeuten, 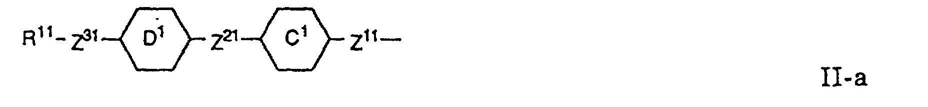 worin
Ringe C¹ und D¹ Pyridin-2,5-diyl, Pyrimidin-2,5-diyl oder ein gegebenenfalls mit Fluor substituiertes 1,4-Phenylen;
Z¹¹ -CH₂CH₂-, -CH₂O-, -COO- oder -OOC-;
Z²¹ eine Einfachbindung,-CH₂O-, -COO-, -OOC-;
Z³¹ -(CH₂)_{m'}-, -(CH₂)_{m'}O-, -(CH₂)_{m'}COO- oder -(CH₂)_{m'}OOC-;
m' eine ganze Zahl von 3 bis 12; und
R¹¹ CH₂=CH-COO-, CH₂=C(CH₃)-COO-, CH₂=CH-O-, 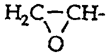
bedeuten.

5. Verbindungen gemäss Anspruch 4 der Formel 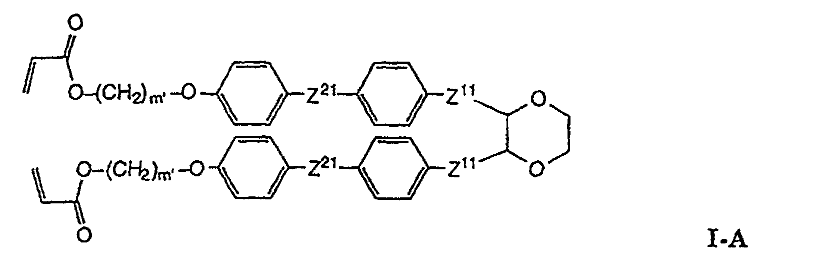 worin
Z¹¹ -COO-;
Z²¹ eine Einfachbindung oder -COO-, insbesondere -COO-;
m' eine ganze Zahl von 3 bis 12 bedeuten.

6. Verbindungen gemäss Anspruch 4 der Formel 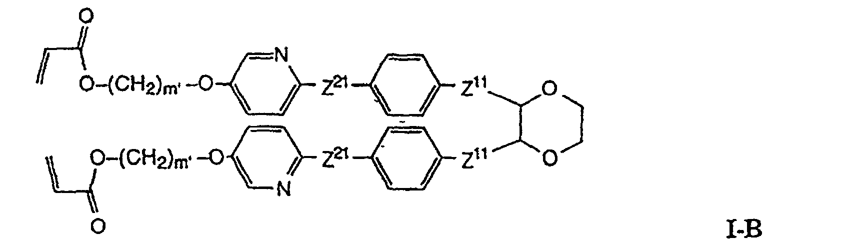 worin
Z¹¹ -COO-;
Z²¹ eine Einfachbindung oder -COO-, insbesondere -COO- ;
m' eine ganze Zahl von 3 bis 12 bedeuten.

7. Verbindungen gemäss Anspruch 4 der Formel 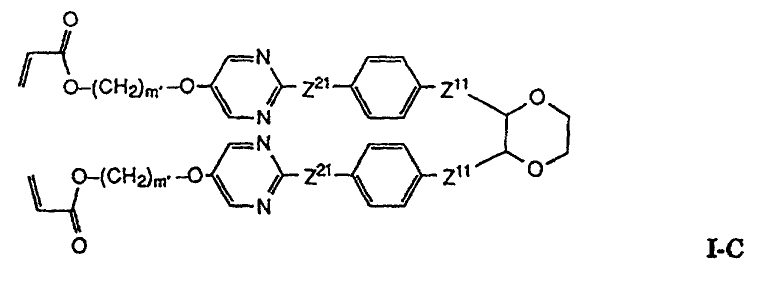 worin
Z¹¹ -COO-;
Z²¹ eine Einfachbindung oder -COO-, insbesondere -COO-;
m' eine ganze Zahl von 3 bis 12 bedeuten.

8. Verbindungen gemäss einem der Ansprüche 5 bis 7, worin Z¹¹ und Z²¹ -COO- bedeuten.

9. Vernetzbare, flüssigkristalline Gemische bestehend aus mindestens 2 Komponenten, wovon mindestens eine Komponente eine Verbindung der in Anspruch 1 definierten Formel I ist.

10. Vernetzbare, flüssigkristalline Gemische gemäss Anspruch 9, **dadurch gekennzeichnet, dass** sie neben einer oder mehreren Verbindungen der Formel I, eine oder mehrere Verbindungen aus der Gruppe der Formeln 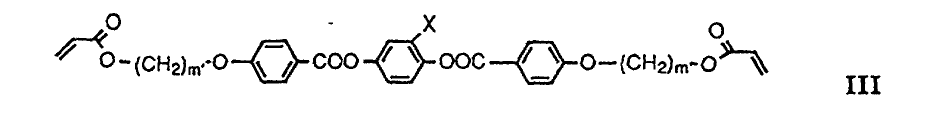 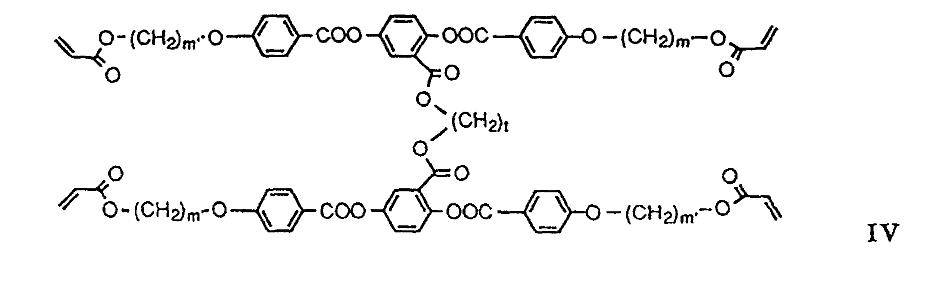 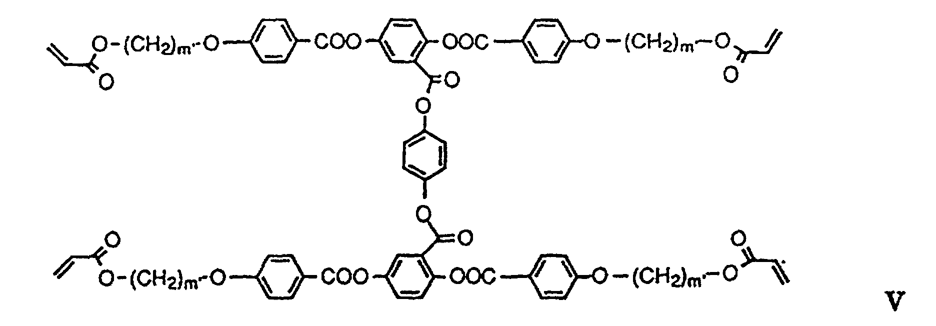 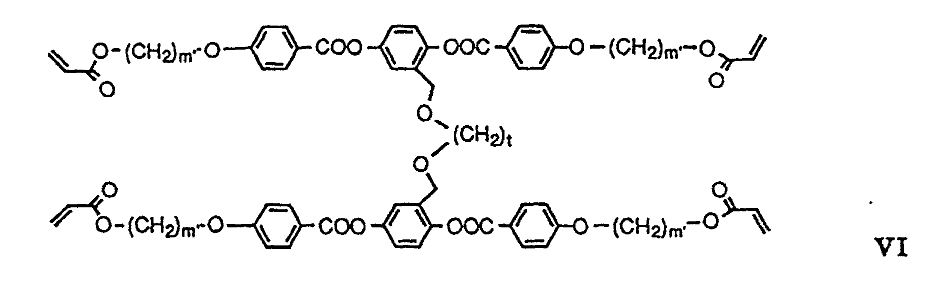 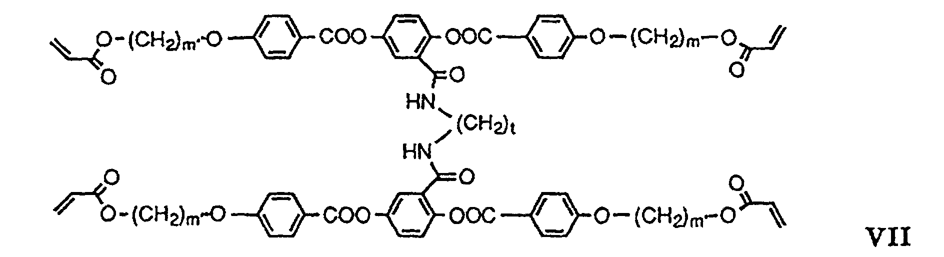 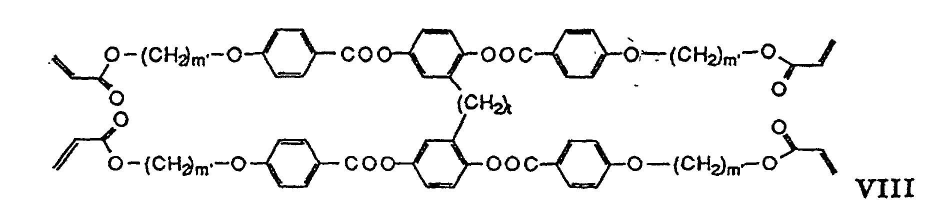 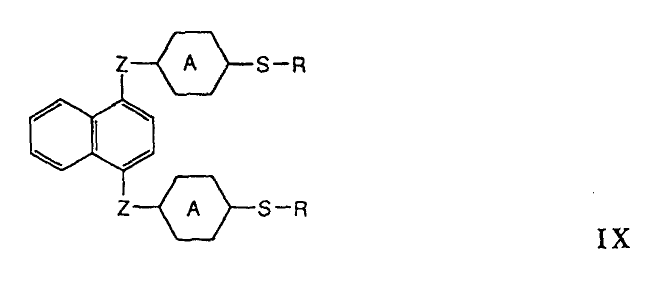 und 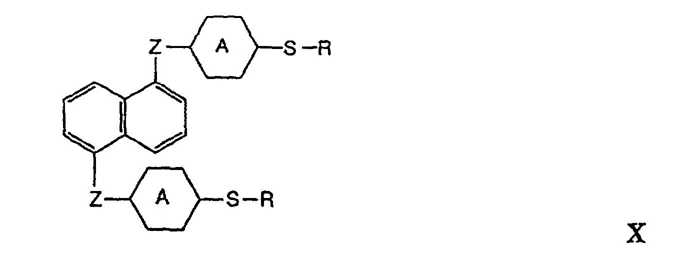 worin
X Wasserstoff, Fluor; Chlor, Brom oder Methyl;
m' eine ganze Zahl von 3 bis 12;
t eine ganze Zahl von 2 bis 12;
Z -OCH₂- oder -OOC-;
A 1,4-Phenylen oder 2- bzw. 3-Fluor-1,4-phenylen;
S -(CH₂)_{m'}- oder -(CH₂)_{m'}O-; und
R CH₂=CH-COO-, CH₂=C(CH₃)-COO-, CH₂=CH-O-, 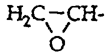
bedeuten.

11. Verwendung von Verbindungen gemäss einem der Ansprüche 1 bis 8 in ihrem vernetzten Zustand für optische Bauelemente.

12. Verwendung von vernetzbaren flüssigkristallinen Gemischen gemäss einem der Ansprüche 9 oder 10 in ihrem vernetzten Zustand für optische Bauelemente.

## Claims

1. A compound of the general formula I 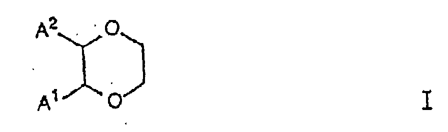 wherein A¹ and A² are mesogenic residues of general formula II 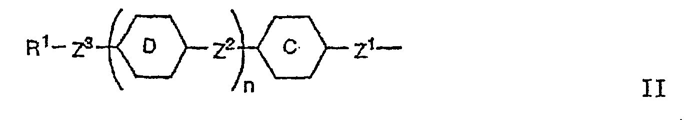 wherein
rings C and D each independently are pyridine-2,5-diyl, pyrimidine-2,5-diyl, trans-1,4-cyclohexylene, trans-1,3-dioxane-2,5-diyl, or 1,4-phenylene unsubstituted or substituted with halogen, methyl or cyan ;
Z¹ is -(CH₂)-(CH₂)ₘ-, -(CH₂)ₘO-, -O(CH₂)ₘ-, -COO-, -OOC-, -(CH₂)ₘCOO- or -(CH₂)ₘOOC- ;
Z² is a single bond, -CH₂-CH₂-, -CH₂O-, -OCH₂-, -COO-, -OOC-, -(CH₂)₄-, -O(CH₂)₃- or -(CH₂)₃O- ;
Z³ is -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, or -NHCH₂(Si[(CH₃)₂]O)ₘSi[(CH₃)₂]CH₂NH- ;
Y is hydrogen or fluorine ;
n is 0, 1 or 2 ;
m is an integer from 1 to 16 ; and
R¹ are cross-linkable groups with the structure CH₂=CH-, CH₂=CH-COO-, CH₂=C(CH₃)-COO-, CH₂=C(Cl)-COO-, CH₂=C(Ph)-COO-, CH₂=CH-COO-(Ph)-, CH₂=CH-CO-(NH)-, CH₂=C(CH₃)-CONH-, CH₂=C(Cl)-CONH-, CH₂=C(Ph)-CONH-, CH₂=C(COOR')-(CH₂)-COO-, CH₂=CH-O-, CH₂=CH-OOC-, Ph-CH=CH-, CH₃-C(=NR')-, -cis,-trans HOO-CR'=CR'-COO-, 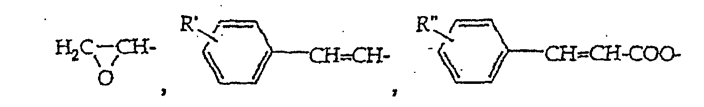
Ph is phenyl;
R' is lower alkyl ;
R" is methyl, methoxy, cyano or halogen;
with the proviso that R¹-Z³ contains no -O-O- or -N-O-.

2. The compound of claim 1, wherein the mesogenic residues A₁ and A₂ are residues of general formula II 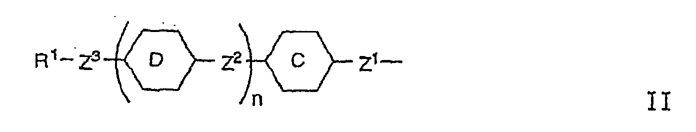 wherein
rings C and D each independently are pyridine-2,5-diyl, pyrimidine-2,5-diyl, trans-1,4-cyclohexylene, trans-1,3-dioxane-2,5-diyl, or 1,4-phenylene unsubstituted or substituted with halogen, methyl and/or cyano ;
Z¹ is -CH₂-CH₂-, -CH₂O-, -COO-, -OOC-, -(CH₂)₄- or -(CH₂)₃O- ;
Z² is a single bond, -CH₂-CH₂-, -CH₂O-, -OCH₂-, -COO-, -OOC-, -(CH₂)₄-, -O(CH₂)₃- or -(CH₂)₃O- ;
Z³ is -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, or -NHCH₂(Si[(CH₃)₂]O)ₘSi[(CH₃)₂]CH₂NH- ;
Y is hydrogene or fluorine;
n is 0 or 1 ;
m is an integer from 1 to 16 ; and
R¹ are cross-linkable groups with the structure CH₂=CH-, CH₂=CH-COO-, CH₂=C(CH₃)-COO-, CH₂=C(Cl)-COO-, CH₂=C(Ph)-COO-, CH₂=CH-COO-(Ph)-, CH₂=CH-CO-(NH)-, CH₂=C(CH₃)-CONH-, CH₂=C(Cl)-CONH-, CH₂=C(Ph)-CONH-, CH₂=C(COOR')-(CH₂)-COO-, CH₂=CH-O-, CH₂=CH-OOC-, Ph-CH=CH-, CH₃-C(=NR')-, -cis,-trans HOO-CR'=CR'-COO-, 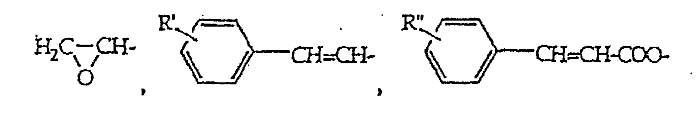
Ph is phenyl;
R' is lower alkyl ;
R" is methyl, methoxy, cyano or halogen;
with the proviso that R¹-Z³ contains no -O-O- or -N-O-.

3. The compound of claim 1 or 2, wherein the mesogenic residues A₁ and A₂ have the same meaning.

4. The compound of claim 3, wherein the residues A₁ and A₂ are a residues of the formula II-a 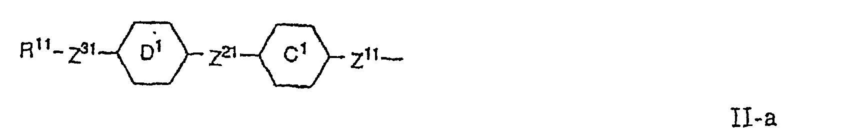 wherein
rings C¹ and D¹ are pyridine-2,5-diyl, pyrimidine-2,5-diyl or 1,4-phenylene optionally substituted with fluorine ;
Z¹¹ is -CH₂-CH₂-, -CH₂O-, -COO- or -OOC- ;
Z²¹ is a single bond, -CH₂O-, -COO-, -OOC- ;
Z³¹ is -(CH₂)_{m'}-, -(CH₂)_{m'}O-, -(CH₂)_{m'}COO- or -(CH₂)_{m'}OOC- ;
m' is an integer from 3 to 12 ; and
R" is CH₂=CH-COO-, CH₂=C(CH₃)-COO-, CH₂=CH-O-,

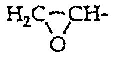

5. The compound of claim 4 having the formula, 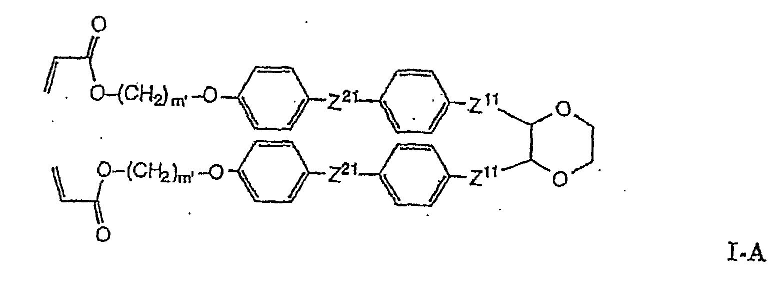 wherein
Z¹¹ is -COO- ;
Z²¹ is a single bond or -COO-, especially -COO- ;
m' is an integer from 3 to 12.

6. The compound of claim 4 having the formula, 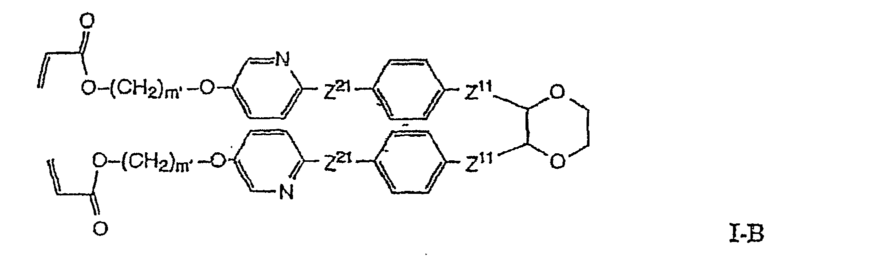 wherein
Z¹¹ is -COO-;
Z²¹ is a single bond or -COO-, especially -COO-;
m' is an integer from 3 to 12.

7. The compound of claim 4, having the formula 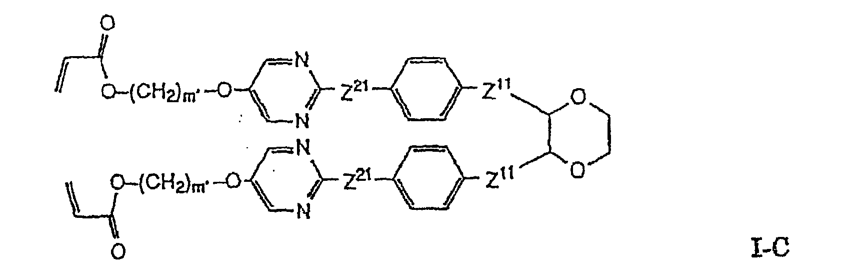 wherein
Z¹¹ is -COO- ;
Z²¹ is a single bond or -COO-, especially -COO- ;
m' is an integer from 3 to 12.

8. Compounds according to one of claims 5 to 7, wherein Z¹¹ and Z²¹ are -COO-.

9. A cross-linkable liquid crystalline mixture comprising at least 2 components, wherein at least one component is a compound having the formula I.

10. The cross-linkable liquid crystalline mixture of claim 9, **characterized in that** it comprises beside one or more compounds having the formula I, one or more of the compounds being part of the formula 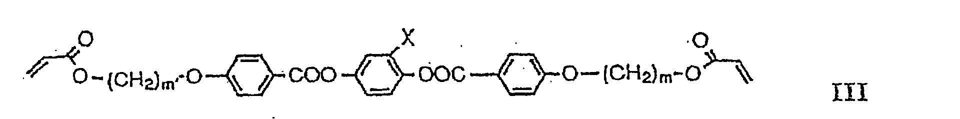 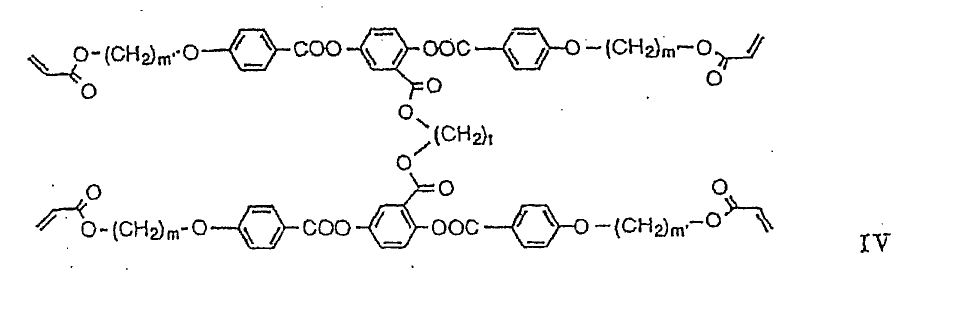 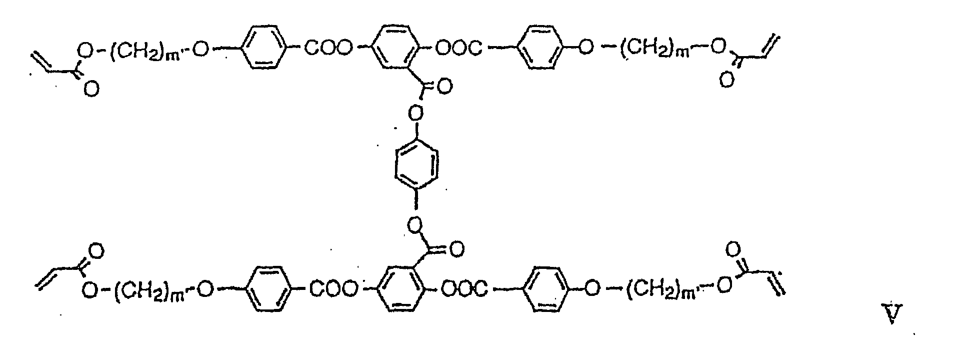 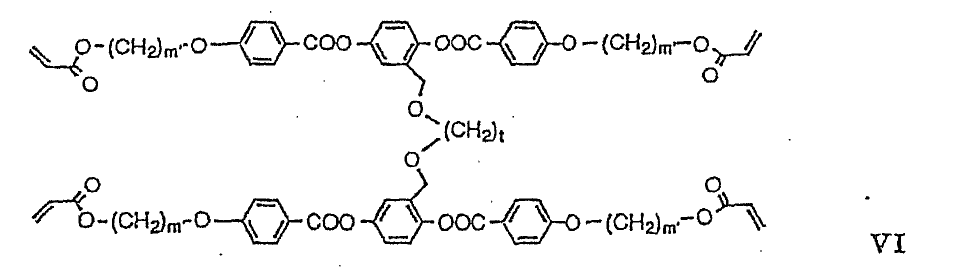 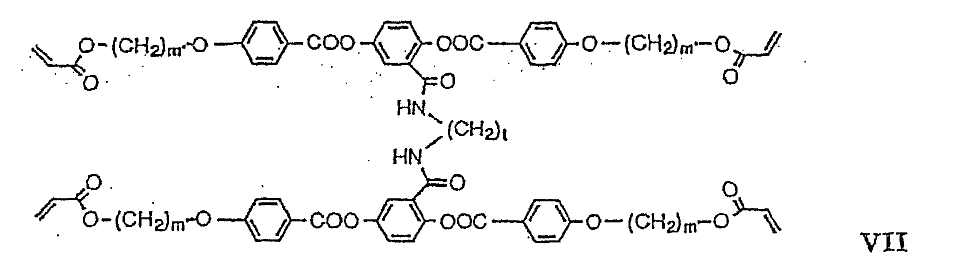 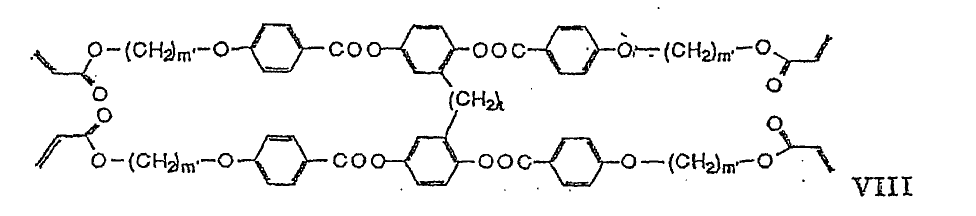 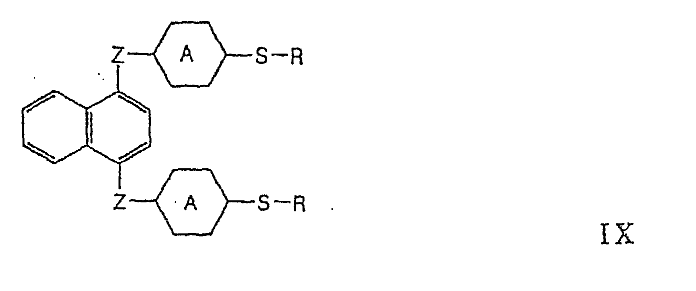 and 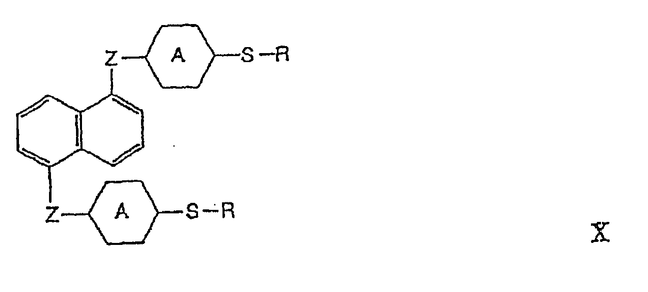 wherein
x is hydrogen, fluorine, chlorine, brome or methyl ;
m' is an integer from 3 to 12 ;
t is an integer from 2 to 12 ;
Z is -OCH₂- or -OOC- ;
A is 1,4-phenylene or 2- or 3-fluoro-1,4-phenylene ;
S is -(CH₂)_{m'}- or -(CH₂)_{m'}O- ; and
R is CH₂=CH-COO-, CH₂=C(CH₃)-COO-, CH₂=CH-O-,

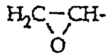

11. Use of compounds according to one of claims 1 to 8 in their cross-linked state for optical components.

12. Use of liquid crystalline mixtures according to one of claims 9 or 10 in their cross-linked state for optical components.

## Revendications

1. Composés de formule générale I 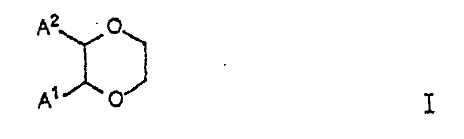 dans laquelle
A₁ et A₂ sont des groupes mésogènes de formule générale II 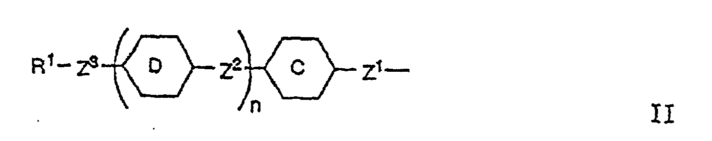 dans laquelle
les cycles C et D représentent, indépendamment l'un de l'autre, un groupe pyridine-2,5-diyle, pyrimidine-2,5-diyle, trans-1,4-cyclohexylène, trans-1,3-dioxane-2,5-diyle, ou un groupe 1,4-phénylène éventuellement substitué par halogéno, méthyle et/ou cyano ;
Z¹ représente un groupe -(CH₂)-(CH₂)ₘ-, -(CH₂)ₘO-, -O(CH₂)ₘ-, -COO-, -OOC-, -(CH₂)ₘCOO- ou -(CH₂)ₘOOC- ;
Z² représente une liaison simple, un groupe -CH₂-CH₂-, -CH₂O-, -OCH₂-, -COO-, -OOC-, -(CH₂)₄-, -O(CH₂)₃- ou -(CH₂)₃O- ;
Z³ représente un groupe -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, ou -NHCH₂(Si[(CH₃)₂]O)ₘSi[(CH₃)₂]CH₂NH- ;
Y représente un atome d'hydrogène ou de fluor ;
n est égal à 0, 1 ou 2 ;
m est égal à un nombre entier de 1 à 16 ; et
R¹ représente des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-(Ph)-, CH₂=CH-CO-(NH)-, CH₂=C(CH₃)-CONH-, CH₂=C(Cl)-CONH-, CH₂=C(Ph)-CONH-, CH₂=C(COOR')-(CH₂)-COO-, CH₂=CH-O-, CH₂=CH-OOC-, Ph-CH=CH-, CH₃-C(=NR')-, -cis,-trans HOO-CR'=CR'-COO-, 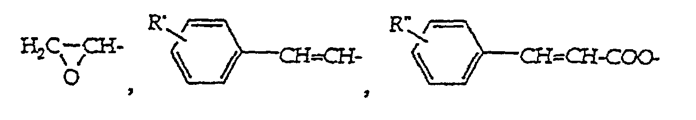
Ph représente un groupe phényle ;
R' représente un groupe alkyle inférieur ;
R" représente un groupe méthyle, méthoxy, cyano ou halogéno ;
étant entendu que R¹-Z³ ne contiennent pas de groupe -O-O- ou -N-O-.

2. Composés selon la revendication 1, où les groupes mésogènes A₁ et A₂ sont des groupes de formule générale II 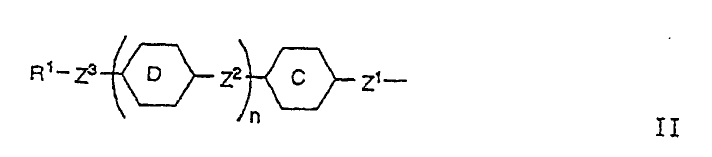 dans laquelle
les cycles C et D représentent, indépendamment l'un de l'autre, un groupe pyridine-2,5-diyle, pyrimidine-2,5-diyle, trans-1,4-cyclohexylène, trans-1,3-dioxane-2,5-diyle, ou un groupe 1,4-phénylène éventuellement substitué par halogéno, méthyle et/ou cyano ;
Z¹ représente un groupe -CH₂-CH₂-, -CH₂O-, -COO-, -OOC-, -(CH₂)₄- ou -(CH₂)₃O- ;
Z² représente une liaison simple, un groupe -CH₂-CH₂-, -CH₂O-, -OCH₂-, -COO-, -OOC-, -(CH₂)₄-, -O(CH₂)₃- ou -(CH₂)₃O- ;
Z³ représente un groupe -(CY₂)ₘ-, -O(CY₂)ₘ-, -(CY₂)ₘO-, -(CY₂)ₘCOO-, -(CY₂)ₘOOC-, -(Si[(CH₃)₂]O)ₘ-, -OCH₂(Si[(CH₃)₂]O)ₘSi[(CH₃)₂]CH₂O-, ou -NHCH₂(Si[(CH₃)₂]O)ₘSi[(CH₃)₂]CH₂NH- ;
Y représente un atome d'hydrogène ou de fluor ;
n est égal à 0 ou 1 ;
m est égal à un nombre entier de 1 à 16 ; et
R¹ représente des groupes réticulables de structure CH₂=CH-, CH₂=CH-COO-, CH₂=C(CH₃)-COO-, CH₂=C(Cl)-COO-, CH₂=C(Ph)-COO-, CH₂=CH-COO-(Ph)-, CH₂=CH-CO-(NH)-, CH₂=C(CH₃)-CONH-, CH₂=C(Cl)-CONH-, CH₂=C(Ph)-CONH-, CH₂=C(COOR')-(CH₂)-COO-, CH₂=CH-O-, CH₂=CH-OOC-, Ph-CH=CH-, CH₃-C(=NR')-, -cis,-trans HOO-CR'=CR'-COO-, 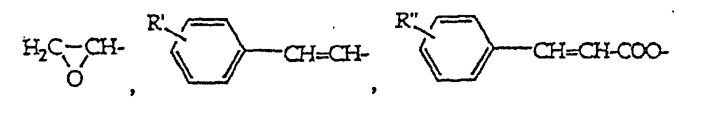
Ph représente un groupe phényle ;
R' représente un groupe alkyle inférieur ;
R" représente un groupe méthyle, méthoxy, cyano ou halogéno ;
étant entendu que R¹-Z³ ne contiennent pas de groupe -O-O- ou -N-O-.

3. Composés selon la revendication 1 ou 2, où les groupes mésogènes A₁ et A₂ ont la même signification.

4. Composés selon la revendication 3, où les groupes A₁ et A₂ représentent un groupe de formule II-a 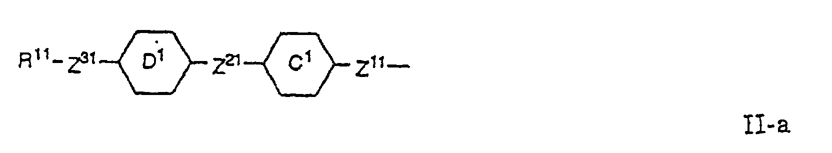 dans laquelle
les cycles C¹ et D¹ représentent un groupe pyridine-2,5-diyle, pyrimidine-2,5-diyle ou un groupe 1,4-phénylène éventuellement substitué par du fluor ;
Z¹¹ représente un groupe -CH₂-CH₂-, -CH₂O-, -COO- ou -OOC- ;
Z²¹ représente une liaison simple, un groupe -CH₂O-, -COO-, -OOC- ;
Z³¹ représente un groupe -(CH₂)_{m'}-, -(CH₂)_{m'}O-, -(CH₂)_{m'}COO- ou -(CH₂)_{m'}OOC- ;
m' représente un nombre entier de 3 à 12 ; et
R¹¹ représente un groupe CH₂=CH-COO-, CH₂=C(CH₃)-COO-, CH₂=CH-O-,

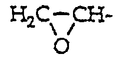

5. Composés selon la revendication 4, de formule 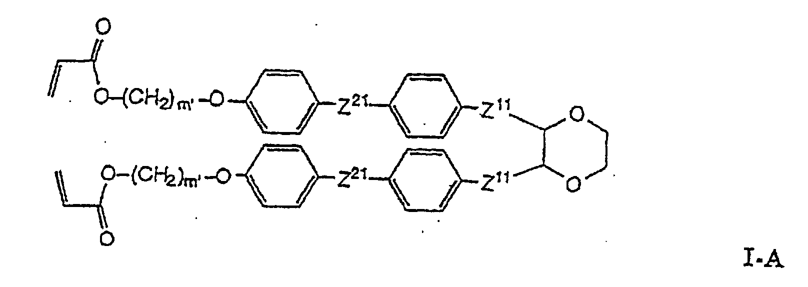 dans laquelle
Z¹¹ représente un groupe -COO- ;
Z²¹ représente une liaison simple ou un groupe -COO-, en particulier un groupe -COO- ;
m' représente un nombre entier de 3 à 12.

6. Composés selon la revendication 4, de formule 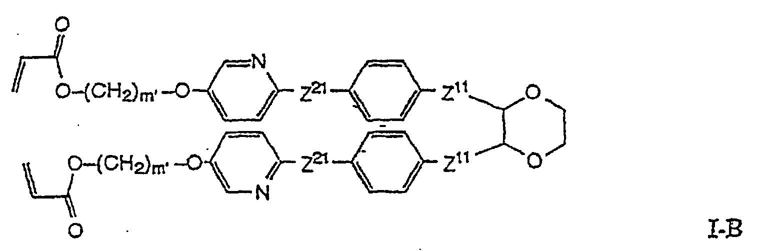 dans laquelle
Z¹¹ représente un groupe -COO- ;
Z²¹ représente une liaison simple ou un groupe -COO-, en particulier un groupe -COO- ;
m' représente un nombre entier de 3 à 12.

7. Composés selon la revendication 4, de formule 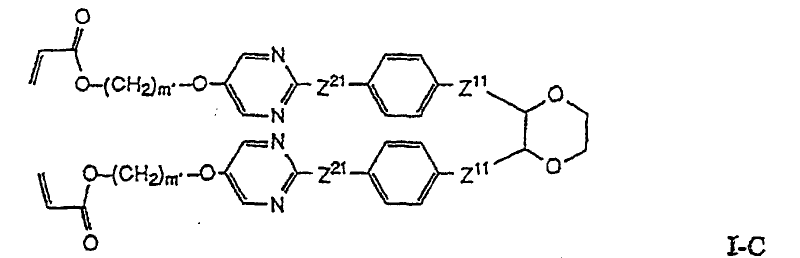 dans laquelle
Z¹¹ représente un groupe -COO- ;
Z²¹ représente une liaison simple ou un groupe -COO-, en particulier un groupe -COO- ;
m' représente un nombre entier de 3 à 12.

8. Composés selon l'une quelconque des revendications 5 à 7, où Z¹¹ et Z²¹ représentent un groupe -COO-.

9. Mélanges réticulables pour cristaux liquides, constitués par au moins 2 composants dont au moins l'un est un composé de formule I telle que définie dans la revendication 1.

10. Mélanges réticulables pour cristaux liquides selon la revendication 9, **caractérisés par le fait qu'**en plus d'un ou plusieurs composés de formule I, ils comprennent un ou plusieurs composés du groupe de formules suivant 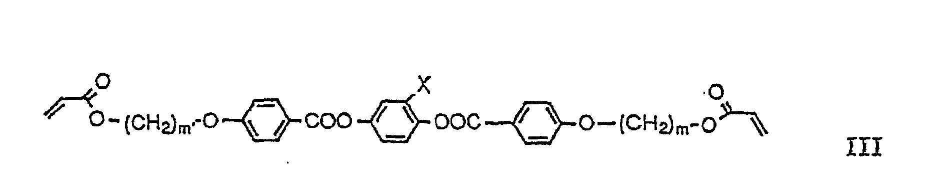 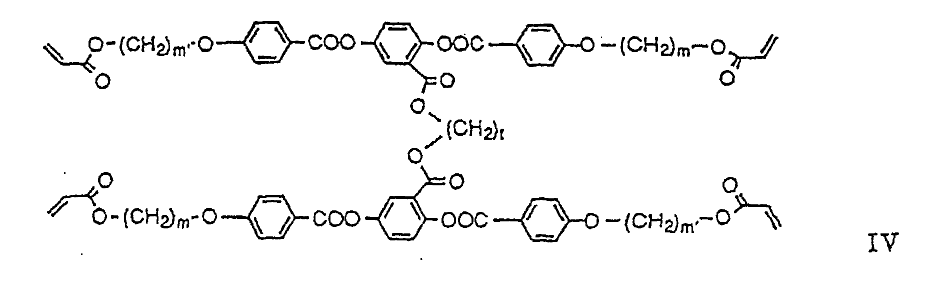 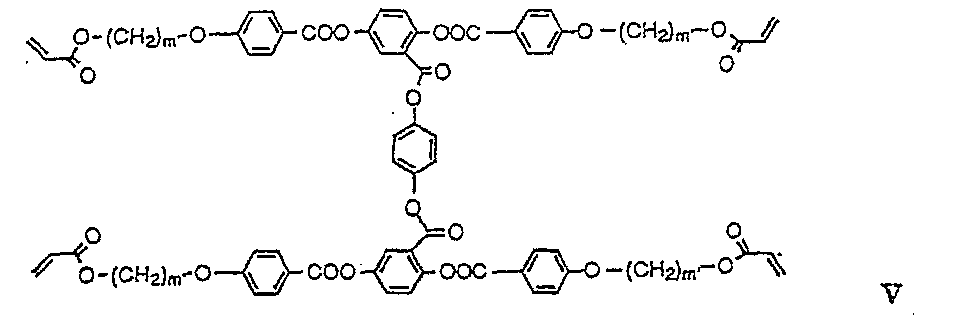 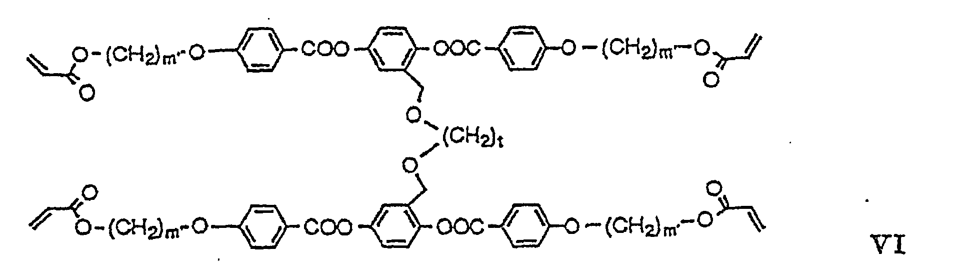 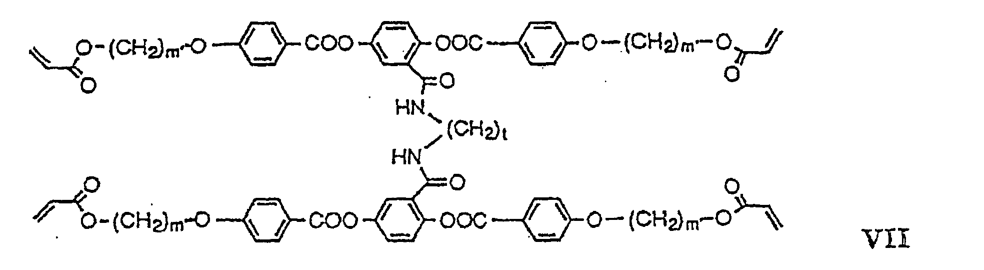 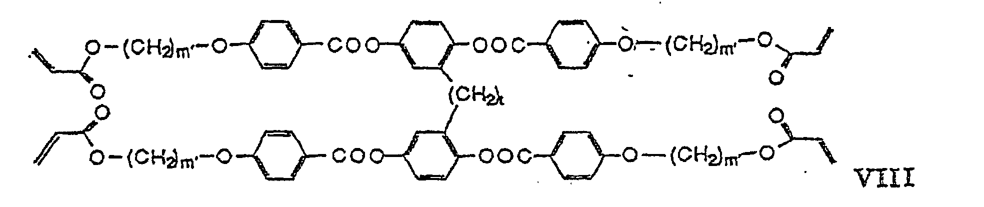 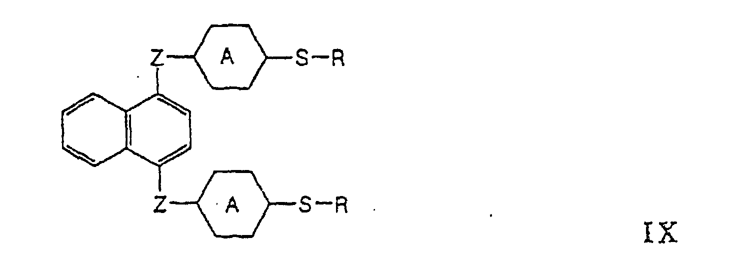 et 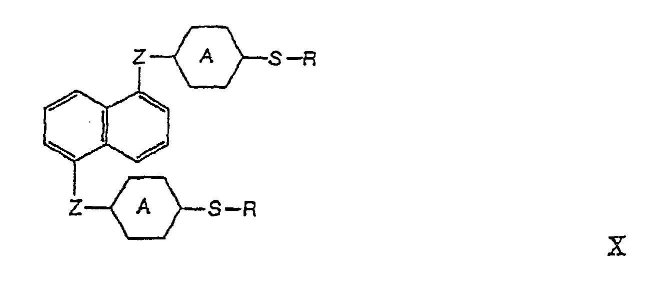 où
x représente un atome d'hydrogène, de fluor, de chlore, de brome ou un groupe méthyle ;
m' représente un nombre entier de 3 à 12 ;
t représente un nombre entier de 2 à 12 ;
Z représente -OCH₂- ou -OOC- ;
A représente un groupe 1,4-phénylène ou 2- ou 3-fluoro-1,4-phénylène ;
S représente -(CH₂)_{m'}- ou -(CH₂)_{m'}O- ; et
R représente un groupe CH₂=CH-COO-, CH₂=C(CH₃)-COO-, CH₂=CH-O-,

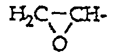

11. Utilisation des composés selon l'une des revendications 1 à 8, à l'état réticulé, pour des composants optiques.

12. Utilisation de mélanges réticulables pour cristaux liquides selon l'une des revendications 9 ou 10, à l'état réticulé, pour des composants optiques.
